# EUROPÄISCHE PATENTANMELDUNG

(11) **EP 2 082 728 A2**
(43) Veröffentlichungstag der Anmeldung: **29.07.2009**
(21) Anmeldenummer: 09150081.9
(22) Anmeldetag: 06.01.2009
(51) Int. Cl.: A61K 8/81, A61K 8/84, A61K 8/86, A61K 8/898, A61Q 5/06

(54) **Verwendung kationischer polymerer Haarfärbemittel**

(30) Priorität: 17.01.2008 DE 102008004974
(71) Anmelder: Beiersdorf AG, 20253 Hamburg (DE)
(72) Erfinder: Klauck, Robert, Dr., 21465 Reinbek (DE); Gutzke, Doreen, 20255 Hamburg (DE); Münchow, Lynn, 22547 Hamburg (DE)

(57) **Zusammenfassung**

Verwendung wenigstens einer der Verbindungen A1 bis A30, A32 bis A36 in einem Kosmetikprodukt zur Färbung von Haaren, insbesondere zur Färbung von menschlichem Kopfhaar, Augenbrauen, Wimpern, Körperbehaarung und/oder Bärten.

## Beschreibung

Die vorliegende Erfindung betrifft neue polymere Farbstoffe und Zusammensetzungen, die diese Verbindungen enthalten, ein Verfahren zu ihrer Herstellung und ihre Verwendung zum Anfärben von organischen Materialien wie keratinhaltigen Fasern, Wolle, Leder, Seide, Cellulose oder Polyamiden.

Schon im vorvergangenen Jahrtausend färbten Menschen ihre Haare. Seit es synthetische Farbstoffe gibt, wurden Haare von viel mehr Menschen gefärbt. Heute färben nach Schätzungen 4/10 der Frauen in Industrieländern ihre Haare. Auch Männer färben.

Synthetische Haarfärbemittel können direktziehende temporäre, direktziehende halbdauerhafte oder dauerhafte Haarfärbemittel sein.

Direktziehende temporäre Haarfärbemittel lassen sich wieder auswaschen.

Halbdauerhafte Haarfärbemittel sollen mehrere Haarwäschen überstehen. Beide Produktgruppen verwenden direktziehende Farbstoffe, die sich in ihrer Affinität zum Haar unterscheiden. Eingesetzt werden Nitrophenylendiamin- oder Nitroamonophenol- sowie Azo- oder Chinoniminfarbstoffe.

Dauerhafte Haarfärbemittel werden auf den Haaren durch chemische Reaktion gebildet, meist durch Oxidation oder oxidative Kupplung.

Zu den Haarfärbemitteln gehören auch Blondiermittel. Diese enthalten z. B. H2O2, Harnstoffperoxid oder Melaminperoxid.

All diese synthetischen Haarfärbemittel weisen Nachteile auf. Oft kommen physiologisch unverträgliche Substanzen oder Komponenten zum Einsatz. Viele Produkte stehen zumindest im Verdacht, Reizungen, allergische Reaktionen oder sonstige Unverträglichkeiten hervorzurufen. Vielfach ist die Anwendung solcher Produkte kompliziert und bleibt Profis vorbehalten. Zudem ist die Anwendungsprozedur oft umständlich und zeitraubend.

Den Nachteilen des Standes der Technik wurde durch die Erfindung abgeholfen. Die Erfindung umfasst die Verwendung wenigstens einer der Verbindungen gemäß der Formeln 1 bis 7 in einem Kosmetikprodukt zur Färbung von Haaren, insbesondere zur Färbung von menschlichem Kopfhaar, Augenbrauen, Wimpern, Körperbehaarung und/oder Bärten. Bekannte Verfahren zum Färben von Augenbrauen und Wimpern haben den Nachteil, dass chemische Reaktionen direkt am empfindlichen Auge stattfinden. Mit den erfindungsgemäßen Färbemitteln ist es möglich, ohne diesen Nachteil intensive und lang anhaltende Färbungen von Augenbrauen und Wimpern zu erzielen.

Die Farbstoffe lassen sich auch in Formulierungen einsetzen, die auf den Wimpern verbleiben und dadurch einen Farbeffekt erzielen. Vorzugsweise werden zu diesem Zweck die Farbstoffe kombiniert mit anderen in der dekorativen Kosmetik üblichen Farbstoffen und Pigmenten eingesetzt. Aufgrund des schonenden Färbeprozesses sind die erfindungsgemäßen Färbemittel besonders gut zum Färben von Körperbehaarung wie Intim-, Brust-, Achselbehaarung sowie von Bärten geeignet.

Besonders intensive und leuchtende Farbergebnisse werden erzielt, in dem man die Haare in einem ersten Verfahrensschritt mit einem aufhellenden Mittel behandelt, dieses nach einer Einwirkzeit von 1 min bis 60 min vorzugsweise 30 bis 40 min abspült und anschließend mit einem Mittel behandelt, dass einen oder mehrere der erfindungsgemäßen Farbstoffe enthält.

Bevorzugt sind zusätzlich Direktfarbstoffe enthalten. Die Farbstoffe eignen sich in besonderer Weise für die Kombination mit in der Kosmetik bekannten Direktfarbstoffen. Dazu eignen sich insbesondere kationische Farbstoffe, aber auch neutrale und anionische Farbstoffe können mit den erfindungsgemäßen Farbstoffen kombiniert werden.

Bevorzugt sind zusätzlich oxidative Farbstoffe enthalten. Oxidative Haarfarbstoffe besitzen oftmals den Nachteil, dass nicht alle Farbrichtungen mit guter Intensität, Waschbeständigkeit und Lichtechtheit gefärbt werden können. Um diesen Mangel auszugleichen, werden üblicherweise oxidative Haarfarbstoffe mit direktziehenden Farbstoffen kombiniert. Aufgrund ihrer hervorragenden Waschbeständigkeit und Lichtechtheit eignen sich die erfindungsgemäßen Farbstoffe in besonderer Weise dazu, mit oxidativen Farbstoffen kombiniert zu werden. Dabei können diese sowohl in der oxidativen Formulierung vorliegen, als auch in einer zweiten Formulierung, die direkt vor der Anwendung zugemischt wird.

Bevorzugt sind zusätzlich Pigmente enthalten. Bevorzugt sind zusätzlich alkalische Streckmittel enthalten. Die erfindungsgemäßen Farbstoffe können auch in alkalischen Streckmitteln eingesetzt werden, wie sie in der Regel für Afro-Haare Verwendung finden.

Bevorzugt sind zusätzlich aufhellende Mittel enthalten. Es wurde außerdem gefunden, dass besonders leuchtende und intensive Farbergebnisse erzielt werden können, indem man das einen oder mehrere erfindungsgemäße Farbstoff enthaltende Mittel vor der Anwendung mit einem die Haare aufhellenden Mittel mischt. Dabei kann das aufhellende Mittel ein Oxidationsmittel wie Wasserstoffperoxid oder Alkali-, Ammonium- oder Erdalkalisalze der Peroxodisulfate enthalten.

Die erfindungsgemäßen Farbstoffe können auch direkt in kosmetischen Mitteln eingesetzt werden, die Oxidationsmittel enthalten. Dabei kann es sich um Gele, Emulsionen, Pasten oder pulverförmige Formulierungen handeln. Besonders anwenderfreundlich und risikoarm sind Färbemittel, die einen deutlichen Effekt erst nach wiederholter Anwendung zeigen. Das gilt besonders zur Abdeckung von grauen Haaren. Durch die hohe Waschbeständigkeit und das schonende Färbeverfahren sind die erfindungsgemäßen Farbstoffe hervorragend für diese Anwendung geeignet.

Bevorzugt sind zusätzlich reduzierende Wellmittel enthalten. Oftmals ist der Wunsch nach einer gleichzeitigen Veränderung der Farbe und der Form der Haare vorhanden. Üblich ist dabei in einem ersten Verfahrensschritt die Anwendung eines reduzierenden Wellmittels und im zweiten Schritt das Auftragen einer oxidativen Haarfarbe. Dieses Verfahren besitzt den Nachteil einer verlängerten Einwirkzeit. Die erfindungsgemäßen Farbstoffe können bereits im Wellmittel eingesetzt werden, so dass zusätzliche Einwirkzeit entfällt. Das Verfahren ist ebenfalls zum Glätten von gewellten Haaren geeignet. Die Erfindung umfasst Verwendungen, bei denen das Produkt als Gel, Emulsion, Paste oder pulverförmige Zubereitung vorliegt. Die erfindungsgemäßen Farbstoffe können in allen zum Ändern der Farbe von Haaren geeigneten Formulierungstypen eingesetzt werden. Dazu zählen Emulsionen, Gele, Shampoos, Pumpschäume, Aerosolschäume, Pulver, Lotionen, Haarwässer sowie Mascaras.

Die Erfindung umfasst auch Verwendungen, bei denen das Produkt in Tuben Flaschen, Beutel, Dosen verpackt vorliegt. Die erfindungsgemäßen Haarfärbemittel können in Tuben, Flaschen, Beutel, Dosen abgefüllt werden.

Besonders geeignet sind Anwendungshilfen, die an der jeweiligen Verpackung angebracht werden können und dem Anwender ein einfaches, sicheres und gleichmäßiges Auftragen des Färbemittels ermöglichen. Dazu zählen Auftragespitzen, Kämme, Bürsten und Schwämme.

Sollen erfindungsgemäße Färbemittel vor der Anwendung mit einem zweiten kosmetischen Mittel gemischt werden, so eignen sich besonders Packmittel mit zwei Kammern, deren Inhalt getrennt dosiert werden kann oder in einer Mischvorrichtung beim Entnehmen vermengt wird.

Die erfindungsgemäßen Färbemittel lassen sich auch in automatischen oder manuellen Farbdispensern einsetzen. Für oxidative Haarfarben ist ein solches Gerät bekannt unter dem Namen Sempre Hair Color Mix. Diese Dispenser besitzen ein oder mehrere Behältnisse, in die Färbemittel mit einem oder unterschiedlichen Farbtönen gefüllt werden. Zusätzlich kann in eines oder mehrere dieser Behältnisse auch kosmetische Mittel, die die Haare aufhellen können, gefüllt werden. Mit Hilfe des Dispensers werden dann die gewünschten Mengen an Färbemittel und eventuell aufhellendem Mittel in ein separates Behältnis dosiert. In diesem separaten Behältnis wird durch Mischen das anwendungsfertige Färbemittel hergestellt.

Die Erfindung umfasst auch Verfahren zur Färbung von Haaren umfassend die Schritte
(a) Behandlung des Haars mit einem aufhellenden Mittel,
(b) bedarfsfalls Behandlung des Haars mit einem reduzierenden Wellmittel,
(c) Behandlung des Haars mit einem oben beschriebenen Kosmetikprodukt.

Die vorliegende Erfindung betrifft weiterhin neue polymere Farbstoffe und Zusammensetzungen, die diese Verbindungen enthalten, ein Verfahren zu ihrer Herstellung und ihre Verwendung zum Färben organischer Materialen wie keratinhaltigen Fasern, Wolle, Leder, Seide, Cellulose oder Polyamiden.

Es ist gut bekannt, dass kationische Verbindungen eine gute Affinität zu negativ geladenem Haar haben. Man nutzt diese Eigenschaften, um das Haar mit kleinen Molekülen, aber auch mit Polymeren, in Kontakt zu bringen.

Für die Verwendung als Färbemittel für menschliches Haar wurden zahlreiche kationische polymere Farbstoffe offenbart, zum Beispiel in US 4,228,259, US 4,182,612 oder FR 2 456 764. Diese Literaturstellen lehren, dass die Polymereinheit die kationische Ladung trägt.

Überraschenderweise wurde gefunden, dass sich sehr gute Färberesultate mit polymeren Haarfarbstoffen erzielen lassen, bei denen sich die kationische Ladung in der Farbstoffeinheit befindet.

### Die vorliegende Erfindung betrifft daher polymere Farbstoffe der Formel

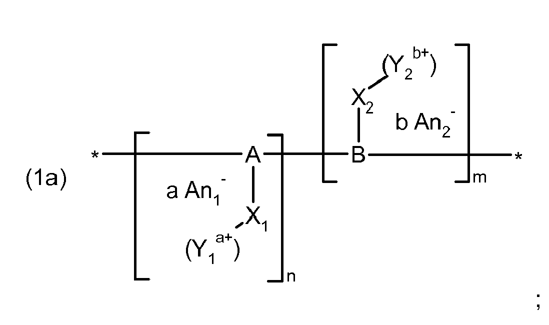

oder 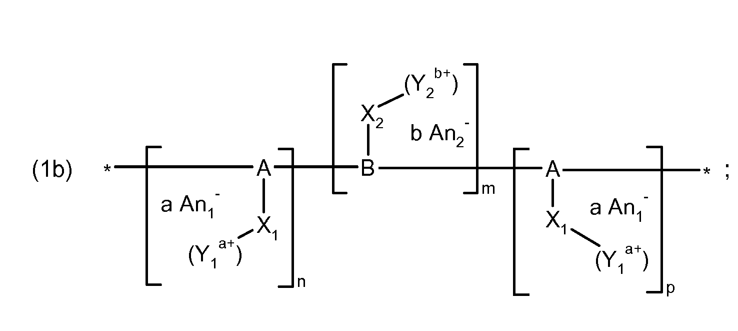 oder 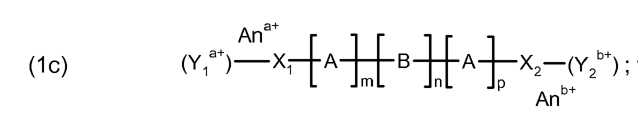 wobei
- A and B: unabhängig voneinander eine Polymerkette darstellen;
- X₁ und X₂: unabhängig voneinander eine Verbindungsgruppe ausgewählt aus -C₁-C₁₀Alkylen-; -C₂-C₁₂Alkenylen-; -C₅-C₁₀Cycloalkylen-; -C₅-C₁₀Arylen; -C₅-C₁₀Arylen-(C₁-C₁₀alkylen)-; -C(O)-; -(CH₂CH₂-O)₁₋₅-; -C(O)O-; -OCO-; -N(R₁)-; -CON(R₁)-; -(R₁)NC(O)-; -O-; -S-; -S(O)-; -S(O)₂-; -S(O)₂-N(R₁R₂)-; oder einer direkten Bindung darstellen;
- R₁ and R₂: unabhängig voneinander Wasserstoff oder unsubstitutiertes oder substituiertes, geradkettiges oder verzweigtes, monocyclisches oder polycyclisches, unterbrochenes oder ununterbrochenes C₁-C₁₄Alkyl; C₂-C₁₄Alkenyl; C₆-C₁₀Aryl; C₆-C₁₀Aryl-C₁-C₁₀alkyl; oder C₅-C₁₀Alkyl(C₅-C₁₀aryl) darstellen;
- Y₁ und Y₂: unabhängig voneinander einen Rest eines organischen Farbstoffs oder Wasserstoff darstellen; wobei wenigstens einer der Reste Y₁ und Y₂ für einen Rest eines organischen Farbstoffs steht;
- An₁, An₂ und An₃: unabhängig voneinander ein Anion darstellen;
- a and b: unabhängig voneinander eine Zahl von 1 to 3 darstellen;
- m: eine Zahl von 0 to 1000 darstellt; und
- n: eine Zahl von 0 bis 1000 darstellt;
- p: eine Zahl von 1 bis 1000 darstellt;
wobei die Summe m + n + p ≥ 3 ist.

C₁-C₁₄Alkyl ist zum Beispiel Methyl, Ethyl, Propyl, Isopropyl, n-Butyl, sek.-Butyl, tert.-Butyl, n-Pentyl, 2-Pentyl, 3-Pentyl, 2,2'-Dimethylpropyl, Cyclopentyl, Cyclohexyl, n-Hexyl, n-Octyl, 1,1',3,3'-Tetramethylbutyl oder 2-Ethylhexyl, Nonyl, Decyl, Undecyl, Dodecyl, Tridecyl oder Tetradecyl.

C₂-C₁₄Alkenyl ist zum Beispiel Allyl, Methallyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, Isododecenyl, n-Dodec-2-enyl oder n-Octadec-4-enyl.

C₆-C₁₀Aryl ist zum Beispiel Phenyl oder Naphthyl.

C₁-C₁₀Alkylen ist zum Beispiel Methylen, Ethylen, Propylen, Isopropylen, n-Butylen, sek.-Butylen, tert.-Butylen, n-Pentylen, 2-Pentylen, 3-Pentylen, 2,2'-Dimethylpropylen, Cyclopentylen, Cyclohexylen, n-Hexylen, n-Octylen, 1,1',3,3'-Tetramethylbutylen, 2-Ethylhexylen, Nonylen oder Decylen.

In den Formeln (1a), (1b) und (1c) sind
Y₁ and Y₂ unabhängig voneinander vorzugsweise ausgewählt aus der Gruppe bestehend aus Anthrachinon-, Acridin-, Azo-, Azamethin-, Hydrazomethin-, Triphenylmethan-, Benzodifuranon-, Cumarin-, Diketopyrrolopyrrol-, Dioxazin-, Diphenylmethan-, Formazan-, Indigoid, Indophenol-, Naphthalimid-, Naphthochinon-, Nitroaryl-, Merocyanin-, Methin-, Oxazin-, Perinon-, Perylen-, Pyrenchinon-, Phthalocyanin-, Phenazin-, Chinonimin-, Chinacridon-, Chinophthalon-, Styryl-, Stilben-, Xanthen-, Thiazin- und Thioxanthenfarbstoffen.

Besonders bevorzugt sind Y₁ and Y₂ unabhängig voneinander ausgewählt aus Azo-, Azomethin-, Hydrazomethin-, Merocyanin-, Methin- and Styrylfarbstoffen.

Am meisten bevorzugt haben Y₁ and Y₂ die gleiche Bedeutung.

Vorzugsweise sind in den Formeln (1a), (1b) und (1c)
A and B unabhängig voneinander ausgewählt aus Polymeren von Monoolefinen and Diolefinen; Mischungen von Polymeren von Monoolefinen und Diolefinen; Copolymeren von Monoolefinen and Diolefinen miteinander oder mit anderen Vinylmonomeren; Polystyrol, Poly(p-methylstyrol), Poly(α-methylstyrol); aromatischen Homopolymeren und Copolymeren, die sich von vinylaromatischen Monomeren ableiten; Copolymeren und hydrierten Copolymeren von vinylaromatischen Monomeren and Comonomeren ausgewählt aus Ethylen, Propylen, Dienen, Nitrilen, Säuren, Maleinsäureanhydriden, Maleimiden, Vinylacetat und Vinylchlorid oder acrylischen Derivaten und Mischungen davon; Pfropfcopolymeren vinylaromatischer Monomere; halogenhaltigen Polymeren; Polymeren, die sich von α,β-ungesättigten Säuren und Derivaten davon ableiten; Copolymeren, die sich von α,β-ungesättigten Säuren und Derivativen davon mit anderen ungesättigten Monomeren ableiten; Polymeren, die sich von ungesättigten Alkoholen und Aminen oder den Acylderivaten oder Acetalen davon ableiten; Homopolymeren und Copolymeren cyclischer Ether; Polyacetalen; Polyphenylenoxiden und -sulfiden, und Mischungen von Polyphenylenoxiden mit Styrolpolymeren oder Polyamiden; Polyurethanen, die sich einerseits von hydroxylterminierten Polyethern, Polyestern oder Polybutadienen und andererseits von aliphatischen oder aromatischen Polyisocyanaten ableiten, sowie Vorstufen davon; Polyamiden und Copolyamiden, die sich von Diaminen und Dicarbonsäuren und/oder von Aminocarbonsäuren oder den entsprechenden Lactamen ableiten; Polyharnstoffen, Polyimiden, Polyamidimiden, Polyetherimiden, Polyesterimiden, Polyhydantoinen and Polybenzimidazolen; Polyestern, die sich von Dicarbonsäuren und Diolen und/oder von Hydroxycarbonsäuren oder den entsprechenden Lactonen ableiten; Polycarbonaten und Polyestercarbonaten; Polyketonen; Polysulfonen, Polyethersulfonen und Polyetherketonen; vernetzten Polymeren, die sich einerseits von Aldehyden und andererseits von Phenolen, Harnstoffen und Melaminen ableiten; Polysiloxanen; natürlichen Polymeren; und Mischungen der erwähnten Polymere.

Beispiele für Polymere von Monoolefinen und Diolefinen sind Polypropylen, Polyisobutylen, Polybut-1-en, Poly-4-Methylpent-1-en, Polyvinylcyclohexan, Polyisopren oder Polybutadien, sowie Polymere von Cycloolefinen, zum Beispiel von Cyclopenten oder Norbornen, Polyethylen (das gegebenenfalls vernetzt sein kann), zum Beispiel High Density Polyethylene (HDPE), High Density and High Molecular Weight Polyethylene (HDPE-HMW), High Density and Ultrahigh Molecular Weight Polyethylene (HDPE-UHMW), Medium Density Polyethylene (MDPE), Low Density Polyethylene (LDPE), Linear Low Density Polyethylene (LLDPE), (VLDPE) und (ULD-PE).

Polyolefine, d.h. die Polymere von im vorstehenden Absatz beispielhaft aufgeführten Monoolefinen, vorzugsweise Polyethylen und Polypropylen, lassen sich durch verschiedene Verfahren herstellen, insbesondere durch die folgenden:
a) radikalische Polymerisation (normalerweise unter hohem Druck und bei erhöhter Temperatur).
b) katalytische Polymerisation unter Verwendung eines Katalysators, der normalerweise ein oder mehr als ein Metall der Gruppen IVb, Vb, Vlb oder VIII des Periodensystems enthält. Diese Metalle haben gewöhnlich einen oder mehrere Liganden, typischerweise Oxide, Halogenide, Alkoholate, Ester, Ether, Amine, Alkyle, Alkenyle und/oder Aryle, die entweder π-oder σ-koordiniert sein können. Diese Metallkomplexe können in freier Form oder fixiert auf Trägern, typischerweise auf aktiviertem Magnesiumchlorid, Titan(III)-chlorid, Aluminiumoxid oder Siliciumoxid, vorliegen. Diese Katalysatoren können im Polymerisationsmedium löslich oder nicht löslich sein. Die Katalysatoren können bei der Polymerisation für sich alleine verwendet werden, oder es können weitere Aktivatoren eingesetzt werden, typischerweise Metallalkyle, Metallhydride, Metallalkylhalogenide, Metallalkyloxide oder Metallalkyloxane, wobei es sich bei den Metallen um Elemente der Gruppen la, IIa und/oder IIIa des Periodensystems handelt. Die Aktivatoren können zweckmäßigerweise durch weitere Ester, Ether, Amin- oder Silylethergruppen modifiziert sein. Diese Katalysatorsysteme werden gewöhnlich als Phillips-, Standard Oil Indiana-, Ziegler-(Natta)-, TNZ-(DuPont)- oder Metallocenkatalysatoren oder als Single Site Catalysts (SSC) bezeichnet.

Mischungen der oben erwähnten Polymere sind zum Beispiel Mischungen von Polypropylen mit Polyisobutylen, von Polypropylen mit Polyethylen (zum Beispiel PP/HDPE, PP/LDPE) und Mischungen verschiedener Typen von Polyethylen (zum Beispiel LDPE/HDPE).

Beispiele für Copolymere von Monoolefinen und Diolefinen miteinander oder mit anderen Vinylmonomeren sind Ethylen-Propylen-Copolymerere, Linear Low Density Polyethylene (LLDPE) und Mischungen davon mit Low Density Polyethylene (LDPE), Propylen-But-1-en-Copolymere, Propylen-Isobutylen-Copolymere, Ethylen-But-1-en-Copolymere, Ethylen-Hexen-Copolymere, Ethylen-Methylpenten-Copolymere, Ethylen-Hepten-Copolymere, Ethylen-Octen-Copolymere, Ethylen-Vinylcyclohexan-Copolymere, Ethylen-Cycloolefin-Copolymere (z.B. Ethylen-Norbornen wie COC), Ethylen-1-Olefin-Copolymere, wobei das 1-Olefin in-situ erzeugt wird; Propylen-Butadien-Copolymere, Isobutylen-Isopren-Copolymere, Ethylen-Vinylcyclohexen-Copolymere, Ethylen-Alkylacrylat-Copolymere, Ethylen-Alkylmethacrylat-Copolymere, Ethylen-VinylacetatCopolymere oder Ethylen-Acrylsäure-Copolymere und ihre Salze (Ionomere) sowie Terpolymere von Ethylen mit Propylen und einem Dien wie Hexadien, Dicyclopentadien oder Ethyliden-Norbornen; und Mischungen solcher Copolymere miteinander und mit den oben erwähnten Polymeren, zum Beispiel Polypropylen-Ethylen-Propylen-Copolymere, LDPE-Ethylen-VinylacetatCopolymere (EVA), LDPE-Ethylen-Acrylsäure-Copolymere (EAA), LLDPE/EVA, LLDPE/EAA und alternierende oder statistische Polyalkylen-Kohlenmonoxid-Copolymere und Mischungen davon mit anderen Polymeren, zum Beispiel Polyamiden.

Die oben erwähnten Homopolymere und Copolymere können eine beliebige räumliche Struktur aufweisen, einschließlich syndiotaktisch, isotaktisch, hemi-isotaktisch oder ataktisch; wobei ataktische Polymere bevorzugt sind. Stereoblock-Polymere sind ebenfalls eingeschlossen.

Beispiele für aromatische Homopolymere und Copolymere, die sich von vinylaromatischen Monomeren einschließlich Styrol ableiten sind α-Methylstyrol, alle Isomere von Vinyltoluol, insbesondere p-Vinyltoluol, alle Isomere von Ethylstyrol, Propylstyrol, Vinylbiphenyl, Vinylnaphthalin, und Vinylanthracen, und Mischungen davon. Homopolymere und Copolymere können eine beliebige räumliche Struktur aufweisen, einschließlich syndiotaktisch, isotaktisch, hemi-isotaktisch oder ataktisch, wobei ataktische Polymere bevorzugt sind. Stereoblock-Polymere sind ebenfalls eingeschlossen.

Beispiele for Copolymere einschließlich der oben erwähnten vinylaromatischen Monomere und Comonomere ausgewählt aus Ethylen, Propylen, Dienen, Nitrilen, Säuren, Maleinsäureanhydriden, Maleimiden, Vinylacetat und Vinylchlorid oder Acrylderivaten und Mischungen davon sind zum Beispiel Styrol-Butadien, Styrol-Acrylnitril, Styrol-Ethylen (Interpolymere), Styrol-Alkylmethacrylat, Styrol-Butadien-Alkylacrylat, Styrol-Butadien-Alkylmethacrylat, Styrol-Maleinsäureanhydrid, Styrol-Acrylnitril-Methylacrylat; Mischungen mit hoher Schlagzähigkeit aus Styrol-Copolymeren und einem anderen Polymer, zum Beispiel einem Polyacrylat, einem Dienpolymer oder einem Ethylen-Propylen-Dien-Terpolymer; und Blockcopolymere von Styrol wie Styrol-Butadien-Styrol, Styrol-Isopren-Styrol, Styrol-Ethylen-Butylen-Styrol oder Styrol-Ethylen-Propylen-Styrol.

Hydrierte aromatische Polymere, die durch Hydrieren der oben erwähnten Polymere gewonnen werden, schließen insbesondere Polycyclohexylethylen (PCHE) ein, das durch die Hydrierung von ataktischem Polystyrol hergestellt und häufig als Polyvinylcyclohexan (PVCH) bezeichnet wird.

Homopolymere und Copolymere können eine beliebige räumliche Struktur aufweisen, einschließlich syndiotaktisch, isotaktisch, hemi-isotaktisch oder ataktisch; wobei ataktische Polymere bevorzugt sind. Stereoblock-Polymere sind ebenfalls eingeschlossen.

Beispiele for Pfropfcopolymere von vinylaromatischen Monomeren sind Styrol oder α-Methylstyrol, zum Beispiel Styrol auf Polybutadien-, Styrol auf Polybutadien-Styrol- oder Polybutadien-Acrylnitril-Copolymeren; Styrol und Acrylnitril (oder Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleimid auf Polybutadien; Styrol und Maleimid auf Polybutadien; Styrol und Alkylacrylate oder Methacrylate auf Polybutadien; Styrol und Acrylnitril auf Ethylen-Propylen-Dien-Terpolymeren; Styrol und Acrylnitril auf Polyalkylacrylaten oder Polyalkylmethacrylaten, Styrol und Acrylnitril auf Acrylat-Butadien- Copolymeren, sowie Mischungen davon mit den unter 6) aufgeführten Copolymeren, zum Beispiel den als ABS-, MBS-, ASA- oder AES-Polymeren bekannten Copolymermischungen.

Beispiele for halogenhaltige Polymere sind Polychloropren, chlorierte Kautschuke, chlorierte und bromierte Copolymere von Isobutylen-Isopren (Halogenbutylkautschuk), chloriertes oder sulfochloriertes Polyethylen, Copolymere von Ethylen und chloriertem Ethylen, Epichlorhydrin-Homo- und -Copolymere, insbesondere Polymere halogenhaltiger Vinylverbindungen, zum Beispiel Polyvinylchlorid, Polyvinylidenchlorid, Polyvinylfluorid, Polyvinylidenfluorid, sowie Copolymere davon wie Vinylchlorid-Vinylidenchlorid-, Vinylchlorid-Vinylacetat- oder Vinylidenchlorid-Vinylacetat-Copolymere.

Beispiele für von α,β-ungesättigten Säuren und Derivaten davon abgeleitete Polymere sind Polyacrylate und Polymethacrylate; Polymethylmethacrylate, Polyacrylamide und Polyacrylnitrile, schlagzäh-modifiziert mit Butylacrylat.

Beispiele für Copolymere der oben erwähnten Monomere miteinander oder mit anderen ungesättigten Monomeren sind Acrylnitril-Butadien-Copolymere, Acrylnitril-Alkylacrylat-Copolymere, Acrylnitril-Alkoxyalkylacrylat- oder Acrylnitril-Vinylhalogenid-Copolymere oder Acrylnitril-Alkylmethacrylat-Butadien-Terpolymere.

Beispiele für von ungesättigten Alkoholen und Aminen oder den Acylderivaten oder Acetalen davon abgeleitete Polymere sind zum Beispiel Polyvinylalkohol, Polyvinylacetat, Polyvinylstearat, Polyvinylbenzoat, Polyvinylmaleat, Polyvinylbutyral, Polyallylphthalat oder Polyallylmelamin; sowie deren Copolymere mit oben erwähnten Olefinen.

Beispiele für Homopolymere und Copolymere cyclischer Ether sind Polyalkylenglykole, Polyethylenoxid, Polypropylenoxid oder Copolymere davon mit Bisglycidylethern.

Beispiele für Polyacetale sind Polyoxymethylen und die Polyoxymethylene, die Ethylenoxid als ein Comonomer enthalten; mit thermoplastischen Polyurethanen, Acrylaten oder MBS modifizierte Polyacetale.

Beispiele für Polyamide und von Diaminen und Dicarbonsäuren und/oder von Aminocarbonsäuren oder den entsprechenden Lactamen abgeleitete Copolyamide sind Polyamid 4, Polyamid 6, Polyamid 6/6, 6/10, 6/9, 6/12, 4/6, 12/12, Polyamid 11, Polyamid 12, aromatische Polyamide ausgehend von m-Xyloldiamin und Adipinsäure; Polyamide hergestellt aus Hexamethylendiamin und Isophthalsäure oder/und Terephthalsäure und mit oder ohne Elastomer als Modifikator, zum Beispiel Poly-2,4,4,-trimethylhexamethylenterephthalamid oder
Poly-m-phenylenisophthalamid; und außerdem Blockcopolymere der oben erwähnten Polyamide mit Polyolefinen, Olefincopolymeren, Ionomeren oder chemisch gebundenen oder gepfropften Elastomeren; oder mit Polyethern, z. B. mit Polyethylenglykol, Polypropylenglykol oder Polytetramethylenglykol; sowie Polyamide oder Copolyamide modifiziert mit EPDM oder ABS; und Polyamide, die während der Herstellung kondensiert wurden (RIM Polyamid-Systeme).

Beispiele für von Dicarbonsäuren und Diolen und/oder von Hydroxycarbonsäuren oder den entsprechenden Lactonen abgeleitete Polyester sind Polyethylenterephthalat, Polybutylenterephthalat, Poly-1,4-dimethylolcyclohexanterephthalat, Polyalkylennaphthalat (PAN) und Polyhydroxybenzoate, sowie von hydroxylterminierten Polyethern abgeleitete Blockcopolyetherester; und außerdem mit Polycarbonaten oder MBS modifizierte Polyester.

Beispiele für einerseits von Aldehyden und andererseits von Phenolen, Harnstoffen und Melaminen abgeleitete vernetzte Polymere sind Phenol-Formaldehyd-Harze, Harnstoff-Formaldehyd-Harze und Melamin-Formaldehyd-Harze.

Beispiele für natürliche Polymere sind Cellulose, Kautschuk, Gelatine und chemisch modifizierte homologe Derivate davon, zum Beispiel Celluloseacetate, Cellulosepropionate und Cellulosebutyrate, oder die Celluloseether, wie Methylcellulose; sowie Kolophonium und Derivate davon.

Beispiele für Mischungen der oben erwähnten Polymere (Polyblends) sind PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylate, POM/MBS, PPO/HIPS, PPO/PA 6.6 und Copolymere, PA/HDPE, PA/PP, PA/PPO, PBT/PC/ABS oder PBT/PET/PC.

Vorzugsweise weisen sowohl die Polymerkette (A und B) und Reste eines organischen Farbstoffs (Y₁ und Y₂) eine funktionelle Gruppe ausgewählt aus der elektrophilen Gruppe ausgewählt aus Halogenid, Tosylat, Mesylat, Methoxy, Säurechlorid, Sulfonylchlorid, Epoxiden, Anhydrid; oder eine nukleophile Gruppe ausgewählt aus Amin, Hydroxy und Thiol auf.

Das Molekulargewicht des polymeren Farbstoffs beläuft sich vorzugsweise auf 400 to 5000.

Mit "Anion" wird zum Beispiel ein organisches oder anorganisches Anion wie Halogenid, vorzugsweise Chlorid und Fluorid, Sulfat, Hydrogensulfat, Phosphat, Bortetrafluorid, Carbonat, Hydrogencarbonat, Oxalat oder C₁-C₈Alkylsulfat, insbesondere Methylsulfat oder Ethylsulfat, bezeichnet; Anion bezeichnet außerdem Lactat, Format, Acetat, Propionat oder ein komplexes Anion, wie das Zinkchlorid-Doppelsalz.

### Am meisten bevorzugt sind polymere Farbstoffe der Formel

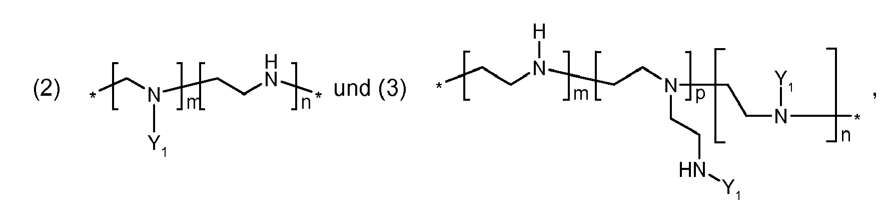

wobei
Y₁ für einen Rest eines organischen Farbstoffs ausgewählt aus Azo-, Anthrachinon-, Azomethin-, Hydrazomethin-, Merocyanin-, Methin- und Styrylfarbstoffen steht; und
m, n und p für eine Zahl von 0 bis 1000 stehen; wobei in Formel (2) die Summe von m und n ≥ 3 und wobei in Formel (3) die Summe von m und n und p ≥ 3.

### Bevorzugt sind außerdem polymere Farbstoffe der Formel

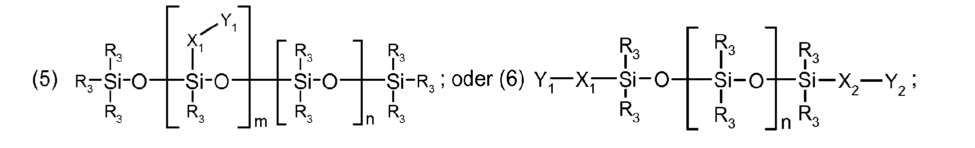

wobei
R₃ für C₁-C₅Alkyl steht; und X₁, X₂, Y₁, Y₂, m und n wie in Formel (1) definiert sind.

### Weiter bevorzugt sind außerdem Farbstoffe der Formel

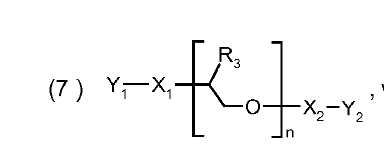

wobei
R₃ für C₁-C₅Alkyl steht; und
X₁, X₂, Y₁, Y₂ und n wie in Formel (1) definiert sind.

Die Erfindung wird durch die nachstehenden Beispiele näher erläutert:
Beispiele für polymere Farbstoffe

### Teil A: Auf Polyethyleniminbasis

Die Beispiele A1 bis A28 wurden hergestellt, indem man eines der Polyethylenimine PEI 1 bis PEI 5 mit einem der Chromophore Chromophor 1 bis Chromophor 5 umsetzt. Die Reaktionsmischungen wurden nach einer der Aufarbeitungsvorschriften 1 bis 5 aufgearbeitet. Die Reaktionsbedingungen und Aufarbeitungsvorschriften für die einzelnen Beispiele sind in Tabelle 1 angeführt. Die analytischen Daten für die Produkte sind in Tabelle 2 angeführt.

In den Beispielen A1 bis A28 verwendete Polyethylenimine:
PEI 1 mit einem zahlenmittleren Molekulargewicht (Mn) von 423 g/mol
PEI 2 mit einem zahlenmittleren Molekulargewicht von 600 g/mol
PEI 3 mit einem zahlenmittleren Molekulargewicht von 1200 g/mol
PEI 4 mit einem zahlenmittleren Molekulargewicht von 1800 g/mol
PEI 5 mit einem zahlenmittleren Molekulargewicht von 10000 g/mol

| In den Beispielen A1 bis A28 verwendete Chromophore: | |
|---|---|
| Chromophor 1: | 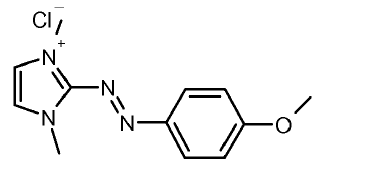 |
| Chromophor 2: | 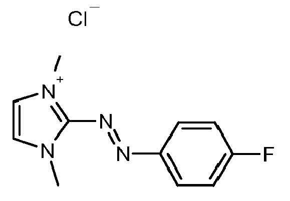 |
| Chromophor 3: | 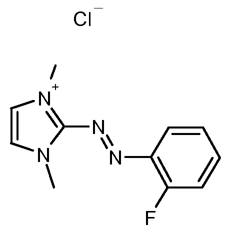 |
| Chromophor 4: | 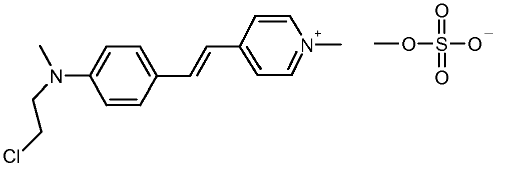 |
| Chromophor 5: | 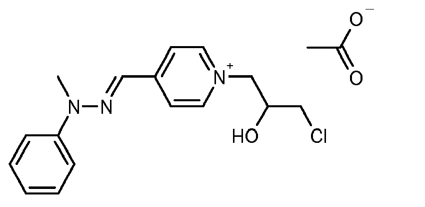 |
| Chromophor 6: | 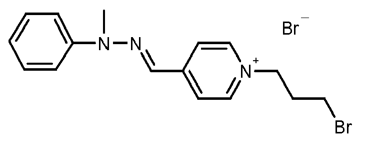 |

### Aufarbeitungsvorschrift 1:

Nach dem Abkühlen wird die Reaktionsmischung zur Trockne eingedampft.
Das Pulver wird nacheinander in Aceton und Dichlormethan aufgenommen, abfiltriert, mit dem gleichen Lösungsmittel gewaschen und getrocknet. Schließlich wird das Produkt in Ethanol gelöst und nochmals getrocknet.

### Aufarbeitungsvorschrift 2:

Die Reaktionsmischung wird auf Raumtemperatur abgekühlt, und das Lösungsmittel wird zur Trockne abgedampft. Das erhaltene Öl wird in Methanol gelöst, und die Lösung wird in Acetonitril getropft. Der Niederschlag wird abfiltriert und im Vakuum getrocknet.

### Aufarbeitungsvorschrift 3:

Die Reaktionsmischung wird auf Raumtemperatur abgekühlt, und das Produkt wird abfiltriert und im Vakuum getrocknet.

### Aufarbeitungsvorschrift 4:

Die Reaktionsmischung wird auf Raumtemperatur abgekühlt und mit 1 Äq. Salzsäure (bezogen auf die Menge an Farbstoff) versetzt. Das ausgefallene Produkt wird abfiltriert und im Vakuum getrocknet.

### Aufarbeitungsvorschrift 5:

Die Reaktionsmischung wird auf Raumtemperatur abgekühlt, und das Lösungsmittel wird zur Trockne abgedampft. Der Rückstand wird in Ethanol gelöst und filtriert. Das Lösungsmittel des Filtrats wird nochmals abgedampft, und das verbliebene Produkt wird mit Isopropanol verrieben und getrocknet.

### Tabelle 1:

Reaktionsbedingungen für die Beispiele A1 bis A28. Mn (PEI) ist das zahlenmittlere Molekulargewicht des Polyethylenimins. Die Chromophoräquivalente sind auf die Anzahl von PEI-Stickstoffatomen bezogen.

| Tabelle 1 | | | | | | | | | |
|---|---|---|---|---|---|---|---|---|---|
| Bei- spiel | Mn(PEI) | Chro- mophor | Äq. Farb- stoff | Lösungs- mittel | T [°C] | Zeit | Aufarbeitungs- vorschrift | Aus- beute | Farbe des Produkts |
| A1 | 423 | Chro- mophor 1 | 0,5 | Isoprop. | 55-60 | 23 h | 1 | 104 | rot |
| A2 | 423 | Chro- mophor 1 | 1 | Isoprop. | 60 | 22 h | 2 | 17 | rot |
| A3 | 423 | Chro- mophor 1 | 1 | Wasser (pH 7-9) | 60 | 50 h | 2 | 15 | rot |
| A4 | 423 | Chro- mophor 1 | 1 | MeOH | 60 | 24 h | 2 | 13 | rot |
| A5 | 423 | Chro- mophor 2 | 0,5 | Isoprop. | RT | 24 h | 3 | 71 | rot |
| A6 | 423 | Chro- mophor 2 | 1 | Isoprop. | RT | 24 h | 3 | 73 | rot |
| A7 | 423 | Chro- mophor 1 | 0,5 | Isoprop. | 60 | 24 h | 4 | 65 | rot |
| A8 | 423 | Chro- mophor 1 | 0,25 | Isoprop. | 60 | 24 h | 4 | 91 | rot |
| A9 | 423 | Chro- mophor 1 | 0,1 | Isoprop. | 60 | 24 h | 4 | 88 | rot |
| A10 | 423 | Chro- mophor 1 | 0,5 | Wasser (pH 11,5) | 40-60 | 24 h | 5 | 58,2 | rot |
| A11 | 423 | Chro- mophor 3 | 0,31 | AcCN | 55 | 24 h | 1 | 130 | violett |
| A12 | 423 | Chro- mophor 3 | 0,5 | AcCN | 55 | 24 h | 1 | 122 | violett |
| A13 | 600 | Chro- mophor 1 | 1 | MeOH | 60 | 22,5h | 2 | 23 | rot |
| A14 | 600 | Chro- mophor 2 | 1 | Isoprop. | 40 | 22 h | 3 | 51 | rot |
| A15 | 600 | Chro- mophor 1 | 0,5 | Isoprop. | 60 | 20,5h | 4 | 23 | rot |
| A16 | 1200 | Chro- mophor 1 | 0,25 | Isoprop. | 55-60 | 24 h | 1 | 105 | rot |
| A17 | 1200 | Chro- mophor 1 | 1 | MeOH | 60 | 24 h | 2 | 23 | rot |
| A19 | 1200 | Chro- mophor 2 | 1 | Isoprop. | 40 | 22 h | 3 | 47 | rot t |
| A19 | 1800 | Chro- mophor 1 | 0,38 | Isoprop. | 55 | | 1 | - | rot |
| A20 | 1800 | Chro- mophor 1 | 1 | MeOH | 60 | 24 h | 2 | 26 | rot |
| A21 | 1800 | Chro- mophor 2 | 1 | Isoprop. | 40 | 22 h | 3 | 45 | rot |
| A22 | 10000 | Chro- mophor 2 | 1 | Isoprop. | 40 | 22 h | 3 | 36 | rot |
| A23 | 423 | Chro- mophor 4 | 0,5 | EtOH | 78 | 193 h | 2 | 56 | orange |
| A24 | 600 | Chro- mophor 5 | 0,5 | BuOH | 60- 100 | 25 h | 4 | 28 | gelb |
| A25 | 600 | Chro- mophor 5 | 0,1 | BuOH | 100 °C | 21 h | 1,92 | 88 | gelb |
| A26 | 600 | Chro- mophor 5 | 0,25 | BuOH | 100 °C | 21 h | 0,86 | 61 | gelb |
| A27 | 423 | Chro- mophor 5 | 0,5 | BuOH | 100°C | 22 h | 0,21 | 12 | gelb |
| A28 | 423 | Chro- mophor 6 | 0,1 | CHCl₃ | 40°C | 6 h | 0,07 | 63,6 | gelb |

**Tabelle 2:**

| Analytische Daten für die Beispiele A1 - A28. Das Verhältnis n(Chromophor)/n(N_{PEI}) ist das Verhältnis von polymergebundenen Chromophormolekülen zur Gesamtzahl von Stickstoffatomen im Polymer. | | | | |
|---|---|---|---|---|
| Beispiel | Mn (GPC- RI) | λₘₐₓ(Wasser) | 1H-NMR (D2O), δ (ppm) | n(Farbstoff)/n(N_{PEI}) |
| A1 | - | 485 nm | 7,9 (m, 2H), 7,5 (m, 2H), 6,9 (m, 2H), 2,6-4,5 (m, 15H), DMSO-d6 | - |
| A2 | 2937 | 490 nm | 6,2-8,1 (br, 6H), 2,6-4,1 (br, 13H) | 0,63 |
| A3 | 1980 | 487 nm | 6,7 (br m, 2H), 7,3 (br m, 2H), 7,8 (br m, 2H) | 0,16 |
| A4 | 3506 | 495 nm | 6,2-8,1 (br, 6H), 2,2-4,9 (br, 13H) | 0,63 |
| A5 | 3145 | 491 nm | 7,7 (m, 2H), 7,3 (m, 2H), 6,8 (m, 1 H), 6,6 (m, 1H), 2,6-4,1 (br, 14H) | 0,48 |
| A6 | 3587 | 493 nm | 7,5 (m, 2H), 7,1 (m, 2H), 6,5 (m, 2H), 2,5-3,7 (br, 13H) | 0,61 |
| A7 | 2491 | 486 nm | 7,8 (m, 2H), 7,3 (m, 2H), 6,7 (m, 2H), 2,6-4,1 (br, 13H) | 0,55 |
| A8 | - | 485 nm | 7,8 (m, 2H), 7,3 (m, 2H), 6,8 (m, 2H), 2,8-3,9 (br, 24H) | 0,23 |
| A9 | - | 488 nm | 7,7 (m, 2H), 7,1 (m, 2H), 6,6 (m, 2H), 2,5-3,9 (br, 49H) | 0,09 |
| A10 | - | 494 nm | 7,9 (br, 2H), 7,3 (br, 2H)6,8 (br, 2H), 4,0 - 2,6 (br, 51 H) | 0,09 |
| A11 | - | 520 nm | - | - |
| A12 | - | 527 nm | 7,8(m, 1 H), 7,5-7,7(m, 3H), 7,15(m, 1 H), 6,85(m, 1 H), 4,1 (m, 6H), 2,6- 3,9(br, 8H), 3,3(s, 3H) | - |
| A13 | 3605 | 488 nm | 6,5-7,5 (br, 4H), 6,1 (m, 2H), 1,9-3,6 (br, 13H) | 0,55 |
| A14 | 4819 | 494 nm | 7,7 (m, 2H), 7,3 (m, 2H), 6,7 (m, 2H), 2,6-4,0 (br, 14H) | 0,51 |
| A15 | 5240 | 485 (Wasser) | 7,5 (m, 2H), 7,1 (m, 2H), 6,5 (m, 2H), 2,5-3,9 (br, 14H) | 0,48 |
| A16 | - | 489 nm | 7,9 (m, 2H), 7,5 (m, 2H), 6,8 (m, 2H), 4,0 (m, 6H), 2,3-3,5 (br, 17H) in Me- | 0,23 |
| | | | OH-d4 | |
| A17 | 5341 | 488 nm | 7,5 (m, 2H), 7,1 (m, 2H), 6,4 (m, 2H), 2,4-3,9 (br, 17H) | 0,4 |
| A18 | - | 491 nm | 7,6 (m, 2H), 7,2 (m, 2H), 6,6 (m, 2H), 2,6-4,0 (br, 16H) | 0,4 |
| A19 | 4670 | - | 7,0-8,1 (br, 4H), 6,5 (m, 2H), 2,4-3,9 (br, 17H) | 0,35 |
| A20 | 5548 | 486 nm | 7,0-8,1 (br, 4H), 6,5 (m, 2H), 2,4-3,9 (br, 19H) | 0,32 |
| A21 | 7264 | 490 nm | 7,6 (m, 2H), 7,2 (m, 2H), 6,6 (m, 2H), 2,5-4,0 (br, 17H) | 0,36 |
| A22 | 11814 | 488 nm | 7,6 (m, 2H), 7,2 (m, 2H), 6,5 (m, 2H), 2,4-4,0 (br, 16H) | 0,42 |
| A23 | 3259 | 452 nm | 8,3 (m, 2H), 7,5 (m, 6H), 6,7 (m, 2H), 4,0 (m, 3H), 2,4-3,6 (br, 22H) | 0,33 |
| A24 | - | 419 nm | 8,5 - 6,9 (br, 10H), 2,8-4,6 (br, 22H) | 0,27 |
| A25 | - | 421 nm | 8,5 - 7,0 (br, 10H), 4,5 - 2 (br, 52H) | 0,1 |
| A26 | - | 420 nm | 8,5-7,0 (br, 10H), 4,6-2,7 (br, 26H) | 0,2 |
| A27 | - | 423 nm | 8,2 - 7,1 (br m, 8H), 6,7 (br m, 2H), 4,0 - 3,2 (br m, 20 H) | 0,28 |
| A28 | - | 419 nm | 8,4 - 6,9 (br, 10H), 4,4 (br, 2H), 3,5 (s, 3H), 3,3 (br, 2H), 2,8 - 2,3 (br, 53H), 2,1 (br,2H) | 0,08 |

### Teil B: Auf Polysiloxanbasis

### Beispiel A-29:

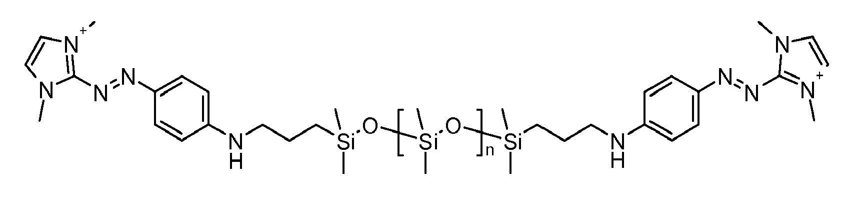

4,75 g Aminopropyl-terminiertes Polydimethylsiloxan ABCR (0,005 mol) werden in 15 ml Isopropanol suspendiert und mit 2,67 g 2-(4-Methoxy-phenylazo)-1,3-dimethyl-3-imidazol-1-ium (Azochromophor A) versetzt. Die Reaktionsmischung wird 24 h auf 55°C erhitzt. Nach dem Abkühlen wird die Reaktionsmischung getrocknet, in 60 ml Chloroform aufgenommen und 3 x mit 40ml Wasser/Essigsäure gewaschen.

Die organische Phase wird nochmals getrocknet, wodurch man 6 g eines roten Pulvers (Ausbeute 81 %) erhält.
NMR in CD2Cl2 in ppm: 7,7, br, 2H; 7,3, br, 2H; 6,8, br 2H; 6,2, br, 1 H; 3,9, s, 6H; 3,25, s, 2H; 1,7, br, 2H; 0,6, br, 2H; 0, br, 62H
λₘₐₓ in MeOH = 518 nm

### Beispiel A-30:

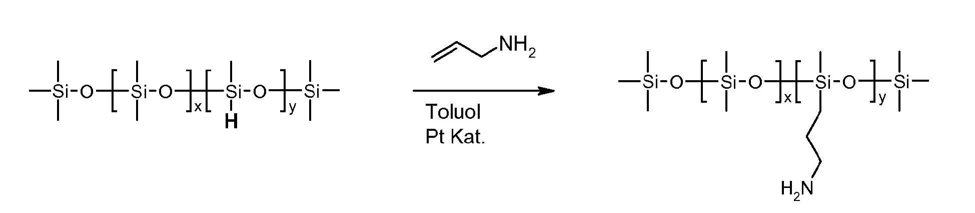

Eine Lösung von 7,5 ml Methylhydrosiloxan-Dimethylsiloxan-Copolymer [68037-59-2] (Gelest HMS 301; MW 1900-2000, 25-30 mol% SiH, d =0,98; 30 mmol-Äq.) in 35 ml Toluol wird mit 2,25 ml Allylamin (30 mmol) und 200 µl Platin(0)-1,3-divinyl-1,1,3,3-tetramethyldisiloxankomplex in Xylol [68478-92-2] versetzt. Die Reaktionsmischung wird 3 Tage lang bei 55°C gerührt und dann zur Trockne eingedampft, wodurch man 8,85 g Polymer erhält.
NMR in DCM in ppm: 2,5 (t, 2H); 1,6 (br , 2H +); 1,4 (br, 2H); 0,4 (t, 2H)

### Beispiel A-31:

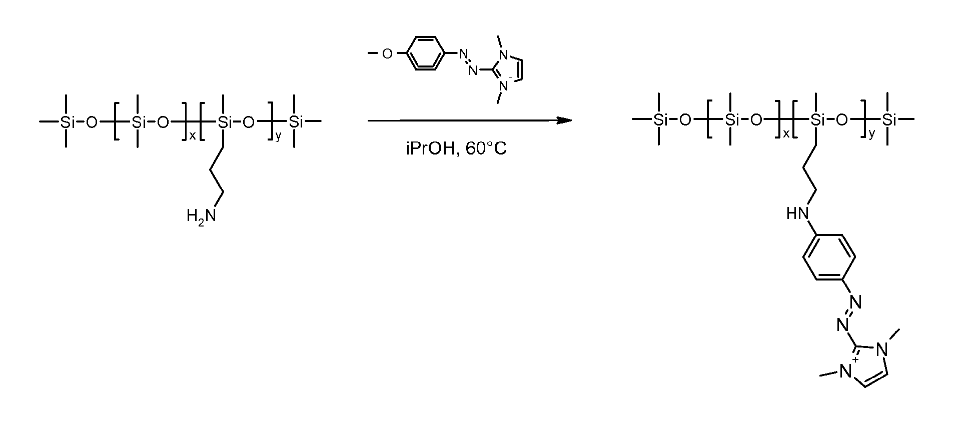

942 mg Polysiloxan A-30 werden in 15 ml Isopropanol aufgenommen und bei 60°C 16 h mit 1,66 g 2-(4-Methoxyphenylazo)-1,3-dimethyl-3-imidazol-1-ium (Azochromophor A) umgesetzt. Nach dem Abdampfen des Isopropanols im Vakuum wird die Reaktionsmischung wieder in 40 ml DCM (2 min in einem Ultraschallbad) und 40 ml Wasser (2 min in einem Ultraschallbad) gelöst. Die DCM/Wasser-Emulsion wird über Nacht stehengelassen. Die DCM- und WasserPhasen werden getrennt, wobei eine zähflüssige Phase zurückbleibt, die nochmals mit DCM und Wasser behandelt wird (gleiche Vorschrift wie oben). Die zähflüssige Phase wird dann in 10 ml Methanol aufgenommen und im Vakuum (1 mbar) getrocknet, wodurch man 596 mg eines roten Feststoffs erhält.
NMR: 1 H in MeOH 7,8 (m, 2H), 7,4 (m, 2H), 6,7 (m, 2H), 3,9 (m, 6H), 3,2 (m, MeOH + 2H), 1,65 (m, 2H), 0 (m, 30H)

### Teil C: Auf Polyetheriminbasis

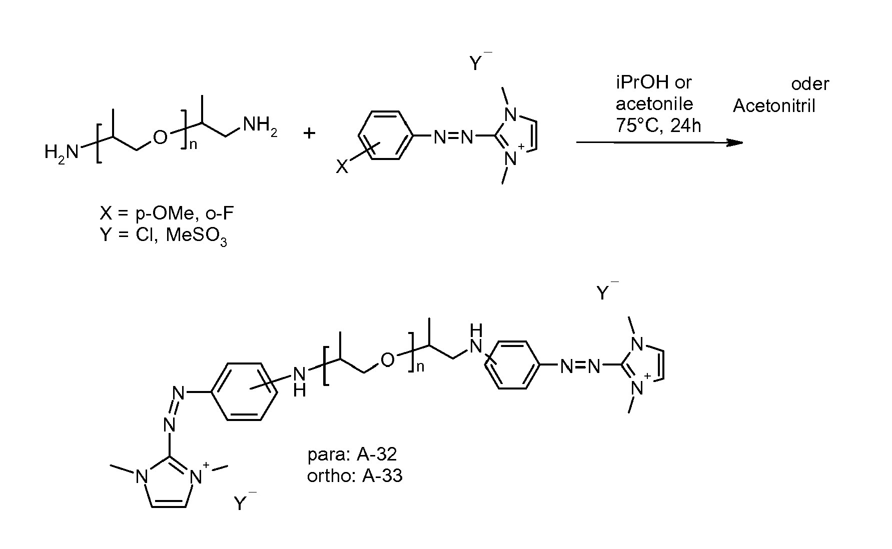

### Beispiel A-32:

Umsetzung von Poly(propylenglykol)bis(2-aminopropylether) mit 2-(4-Methoxyphenylazo)-1,3-dimethyl-3H-imidazol-1-iumchlorid:
2 g (0,0044 Äq.-mol/N) Poly(propylenglykol)bis(2-aminopropylether) (von Aldrich CAS [9046-10-0] MW 456) werden bei 55° bis 80°C 21 h mit 2,66 g (0,01 mol) 2-(4-Methoxyphenylazo)-1,3-dimethyl-3H-imidazol-1-iumchlorid in 14 ml Isopropanol umgesetzt. Nach dem Abkühlen wird die Reaktionsmischung zur Trockne eingedampft, in 100 ml Dichlormethan solubilisiert und 3x mit 25 ml Wasser und Natriumcarbonatlösung gewaschen, wodurch man nach dem Eindampfen der organischen Phase 3,16 g des erwarteten roten Polymers (Ausbeute 68 %) erhielt.
NMR in DMSO in ppm: 7,4, br, 1 H; 7,15, s, 1 H; 6,6, br, 1 H; 3,75, s, 3H; 3,2-3,7, br, 10H; 1,1, br, 9H λₘₐₓ in MeOH = 503 nm

### Beispiel A-33:

Umsetzung von Poly(propylenglykol)bis(2-aminopropylether) mit 2-(2-Fluorphenylazo)-1,3-dimethyl-3H-imidazol-1-iumsulfonat:
1 g (0,0044 Äq.-mol/N) Poly(propylenglykol)bis(2-aminopropylether) (von Aldrich CAS [9046-10-0] MW 456) werden bei 75°C 24 h mit 1,92 g (0,0044 mol) 2-(2-Fluorphenylazo)-1,3-dimethyl-3H-imidazol-1-iumsulfonat in 6 ml Acetonitril umgesetzt. Nach dem Abkühlen wird die Reaktionsmischung zur Trockne eingedampft, in 70 ml Dichlormethan solubilisiert und 3x mit 25 ml Wasser gewaschen, wodurch man nach dem Eindampfen der organischen Phase 1,58 g des erwarteten violetten Polymers (Ausbeute 67 %) erhält.
NMR in CH₂Cl₂ in ppm: 9,3, br 1 H; 8, br, 2H, 7,65, d, 1 H; 7,4, t, 1 H; 6,7, br, 1 H; 4,1, s, 6H; 3,1-3,8, br, 23H; 1,3, br, 5H; 0,9-1,1, br, 18H
λₘₐₓ in MeOH = 531 nm

### Beispiel A-34:

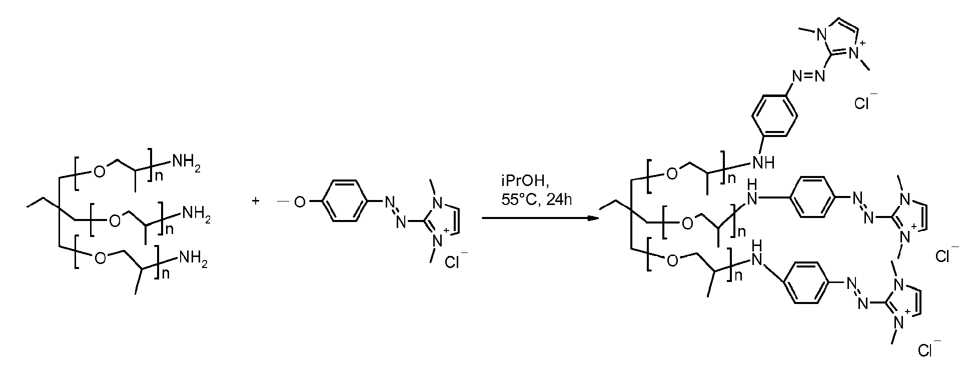

Umsetzung von Trimethloylpropantris[poly(propylenglykol)amin-terminiertem] Ether mit 2-(4-Methoxyphenylazo)-1,3-dimethyl-3H-imidazol-1-iumchlorid:
1 g (0,00631 mol/N) Trimethloylpropantris[poly(propylenglykol)amin-terminierter] Ether von Aldrich CAS [39423-51-3] wird bei 80°C 24 h mit 1,68 g (0,00631 mol) 2-(4-Methoxyphenylazo)-1,3-dimethyl-3H-imidazol-1-iumchlorid in 7 ml Isopropanol umgesetzt. Nach dem Abkühlen wird die Reaktionsmischung zur Trockne eingedampft, in 70 ml Dichloromethan solubilisiert und 3 x mit 25 ml Wasser gewaschen, wodurch man nach dem Eindampfen der organischen Phase 2,23 g des erwarteten roten Polymers (Ausbeute 90 %) erhält.
NMR in CD2Cl2 in ppm: 7,6, br, 3H; 6,8, br, 2H, 6,5, br, 2H; 6,3, s, 2H; 2,6-3,9, br, 25H; 0,6-1,4, br, 14H
λₘₐₓ in MeOH = 518 nm

### Beispiel A-35

Eine Suspension von 0,5 g Copolyglycidylmetacrylat-butylacrylat (1,72 mmol Epoxy-Äq.) in 1 ml Acetonitril und 4 ml Isopropanol wird auf 65°C erhitzt, bis das Polymer dispergiert ist. Dann werden bei 40°C 0,55 g 2-(N,N'-4-[Methyl-(2-methylaminoethyl)amino]phenylazo)-1,3-dimethyl-3H-imidazol-1-iumchlorid (1,72 mmol) zugesetzt, und die Reaktionsmischung wird nochmals 40 h auf 60°C erhitzt.

Nach dem Abkühlen wird die Reaktionsmischung zur Trockne eingedampft und in Kochsalzlösung aufgenommen, und das Polymer wird mit Dichlormethan extrahiert.

Die weißen Salze werden abfiltriert, und die organische Phase wird eingedampft und in Methanol gelöst. Der Rest an Feststoffen wird abfiltriert und die Lösung wird eingedampft, wodurch man 1 g eines dunkelroten Pulvers erhält.

NMR in MeOD in ppm: 7,9; br, 2H; 7,5, s, 2H; 6,95, br, 2H; 4, br, 12H; 3,5-3,9, br, 3H; 3,1-3,3, br, 6H (+MeOD); 2,7, br, 2H; 2,5, br, 2H; 2,4, br, 4H; 1,6, br, 3H; 0-7-1,5, br, 12H

### Beispiel A-36:

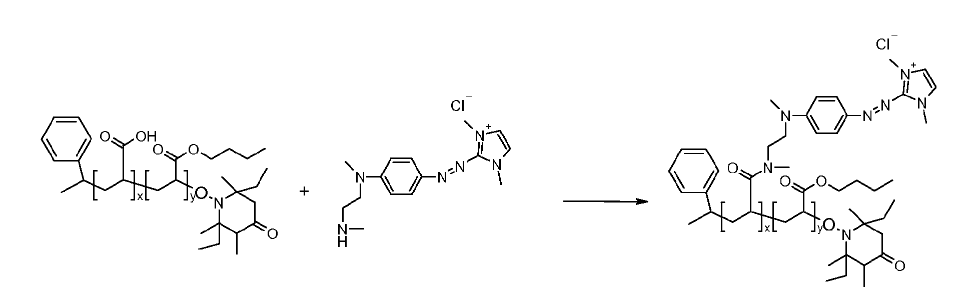

1,06 g Copolyacrylsäure-butylacylat (19,5%ige Lösung in Dioxan (1,6 mmol-Äq. COOH)) werden mit 5 ml Acetonitril verdünnt.

0,5 g N-Ethyl-N'-(3-dimethylamiopropyl)carbodiimid (3,2 mmol) werden zugesetzt, und die Reaktionsmischung wird auf 5°C abgekühlt.

Nach 45 min werden 0,52 g 2-(N,N'-4-[Methyl-(2-methylaminoethyl)amino]phenylazo)-1,3-dimethyl-3H-imidazol-1-iumchlorid (1,6 mmol) und 0,48 g Pyridin (4,8 mmol) zugegeben, und die Reaktionsmischung wird dann 18 h bei Raumtemperatur gerührt.

Nach dem Abdampfen der Lösungsmittel wird das Pulver mit Essigsäureethylester gewaschen und getrocknet, wodurch man 0,74 g eines dunkelroten Pulvers erhält.
NMR in MeOD in ppm: 7,8, br, 2H; 7,5, br, 2H; 6,9, br, 2H; 3,9, br, 8H; 3,6, br, 4H; 2,5-3,4, br, 23H; 0,7-2,0, br, 16H

### Beispiele für kosmetische Zubereitungen

Zur Herstellung der nachfolgenden Rezepturbeispiele wurden folgende Rohstoffe verwendet:

| **Handelsname** | **Lieferant** | **Bezeichnung** |
|---|---|---|
| Eumulgin B1 | Cognis | Ceteareth-12 |
| Lanette O | Cognis | Cetearylalkohol |
| Crotein Q | Croda | Hydroxypropyltrimonium hydrolyzed keratin |
| Magnesia 10 | Magnesia | Magnesiumcarbonat |
| Aerosil 200 | Evonik Degussa | Silica |
| Volpo CS 20 (B) ET2687 | Croda | Ceteareth-20 |
| Dehydol LS3 Deo N | Cognis | Laureth-3 |
| Tegin VS | Evonik-Goldschmidt | Glyceryl stearate SE |
| Comperlan 100 | Cognis | Cocamide MEA |
| Super Hartolan B | Croda | Lanolin alcohol |
| Tego Amid S 18 | Evonik Degussa | Stearamidopropyl Dimethylamine |
| Jellner QH 200 | Daicel | Polyquaternium-10 |
| Euroxide LO | EOC Surfactants | Lauramine oxide |
| Avocado Oil | Henry Lamotte | Persea Gratissima oil |
| Phenoxyethanol | BASF | Phenoxyethanol |
| Benzylalkohol | Merck | Benzylalkohol |
| Euxyl K 300 | Schülke & Mayr | Phenoxyethanol + Methylparaben + Ethylpa- raben + Butylparaben + Propylparaben + Isobutylparaben |
| Neolone 950 | Rohm & Haas | Methylisothiazolinone |
| C Sorbidex NC 16225 | Cargill | Sorbitol |
| Glucquat 125 | Chemron | Lauryl Methyl Gluceth-10 Hydroxypro- pyldimonium Chloride |
| Octopirox | Clariant | Piroctone Olamine |
| Turpinal SL | Thermphos | Etidronic Acid |
| Uantox EBATE | Universal Preserv-A- Chem | Erythorbic Acid |
| EDETA BD | BASF | Disodium EDTA |
| Isopropyl Alcohol | Sasol | Isopropyl Alcohol |
| Propylenglycol | | Propylene glycol |
| Hopfen-Extrakt HS 2367 G | Grau Aromatics | Propylene Glycol + Humulus Lupulus |
| Dow Corning 246 Fluid | Dow Corning | Cyclohexasiloxane |
| Ondina 917 | Shell | Paraffinum liquidum |
| Eutanol G | Cognis | Octyldodecanol |
| Salcare Super 7 | Ciba | Polyquaternium-7 |
| Natrosol 250 HHX pharm | Hercules | Hydroxyethylcellulose |
| Carbopol 981 | Lubrizol | Carbomer |
| Saboderm DO | Polygon | Decyl Oleate |
| Sabosorb MLE | Polygon | Polysorbate-20 |
| Jaguar Excel | Rhodia | Guar Hydroxypropyltrimonium Chloride |
| Genamin KDMP | Clariant | Behentrimonium Chloride |
| Dehyquart A-CA | Cognis | Cetrimonium Chloride |
| Tego-Betain F 50 | Evonik Goldschmidt | Cocamidopropyl Betaine |
| Dow Corning 939 Catio- nic Emulsion | Dow Corning | Amodimethicone + Trideceth-12 + Cetrimo- nium Chloride |
| Texapon N70 | Cognis | Sodium Laureth Sulfate |
| Texapon ALS/IS | Cognis | Ammonium Lauryl Sulfate |
| Miranol Ultra C32 | Rhodia | Sodium Cocoamphoacetate |
| Uvinol DS 49 | BASF | Benzophenone-9 |
| Euperlan PK 900 Benz- W | Cognis | PEG-3 Distearate + Sodium Laureth Sulfate + Benzoic Acid |
| Lamesoft TM Benz | Cognis | Glycol Distearate + Coco Glucoside + Glyc- eryl Oleate + Glyceryl Stearate |
| Kakaobutter, raffiniert Art 1604 | Gustav Heess | Theobroma Cacao |
| Luviskol VA 64 W | BASF | VP/VA Copolymer |
| Gafquat 755N-PW | ISP | Polyquaternium-11 |
| Texapon Z95P | Cognis | Sodium Lauryl Sulfate |
| Crosilk Protein Complex | Croda | Hydrolyzed Silk |
| Nutrilan Keratin W | Cognis | Hydrolyzed Keratin |
| Uvinul A Plus | BASF | Diethylamino Hydroxybenzoyl Hexyl Ben- zoate |
| Solamer GR8 | Nalco | Polyamide-2 |
| Timica Silver Sparkle | BASF | Mica + Titanium Dioxide |
| Keltrol RD | Cp Kelco | Xanthan Gum |
| Silwet Copolymer L- 7600 | Witco | Dimethicone Copolyol |
| Luviquat FC 370 | BASF | Polyquaternium-16 |
| Celquat L-200 | National Starch | Polyquaternium-4 |
| Luviquat Mono CP | BASF | Hydroxyethyl cetyldimonium phosphate |
| Luviquat Supreme | BASF | Polyquaternium-68 |
| Cremophor RH 40 | BASF | PEG-40 Hydrogenated Castor Oil |
| Gelcarin, Type GP 379 | FMC | Chondrus Crispus (carrageenan) |
| Grindsted Guar 178 | Danisco | Guar Gum |
| Coenzyme Q 10 | Kougen Company | Ubiquinone |
| 1,3 Butylene Glycol | Daiichi | Butylene Glycol |
| Pluracol E 8000 | BASF | PEG-150 |
| Dow Corning 245 Fluid | DOW Corning | Cyclomethicone |
| Bienenwachs weiss | Kahl | Cera Alba |
| Carnauba Wax 2442 | Kahl | Cera Carnauba |
| Lunacera C 40 | H.B. Fuller | Candelilla Cera |
| Lunacera MWN | H.B. Fuller | Cera Microcristallina |
| Kester Wachs K82H | Koster Keunen Hol- land | C20-40 Alkyl Stearate |
| Silfoam SE 2 | Wacker-Chemie | Simethicone |
| Resyn XP | National Starch | Acrylates/Octylacrylamide Copolymer |
| Antaron V-220 | International Special- ty Products | VP Eicosene Copolymer |
| Gummi Arabicum, Type 1307 | König & Wiegand | Acacia Senegal |
| Luzenac Pharma 30A | Luzenac | Talc |
| Ariabel Black | Warner-Jenkinson | CI 77499 |
| C61-1245 SunCROMA Chrome Oxide Green | Sun Chemical | CI 77288 |
| Pristerene 4960 | Uniquema | Stearic Acid |
| Fitoderm | Hispano Quimica S. A. | Squalane |
| Meadowfoam Seed oil | Fanning | Limnanthes Alba |
| Covastyle TBQ | LCW | t-Butylhydrochinon |
| Aristoflex AVC | Clariant | Ammonium Acryloyldimethyltaurate/VP Co- polymer |

Zur Herstellung der nachfolgenden Rezepturbeispiele wurden folgende direktziehende Farbstoffe verwendet:

| **Handelsname** | **Bezeichnung** |
|---|---|
| Arianor Steelblue | Basic Blue 99 |
| Arianor Madder Red | Basic Red 76 |
| Arianor Straw Yellow | Basic Yellow 57 |
| Arianor Mahogany | Basic Brown 16 |
| Arianor Sienna Brown | Basic Brown 17 |
| Vibracolor Ruby Red | Basic Red 51 |
| Vibracolor Citrus Yellow | Basic Yellow 87 |
| Vibracolor Flame Orange | Basic Orange 31 |
| HC Blue 2 | HC Blue No.2 |
| HC Red 3 | HC Red No.3 |
| NHEAP | 4-Hydroxyethylamino-3-nitrophenol |
| Rodol 9R | 2-AMINO-6-CHLORO-4-NITROPHENOL |
| HC Violet BS | N,N'-BIS(2-HYDROXYETHYL)-2-NITRO-p-PHENYLENEDIAMINE |
| Patent Blue V | Cl 42051 |
| Brilliant Blue | Cl 42090 |
| Azorubin | Cl 14720 |
| Acid Violet 43 | Cl 60730 |
| Henna Leaves | Cl 75480 |

Zur Herstellung der nachfolgenden Rezepturbeispiele wurden folgende oxidative Zwischenprodukte verwendet:

| **Handelsname** | **Bezeichnung** |
|---|---|
| 2,5-Diaminotoluolsulfat | Toluene-2,5-Diamine |
| Jarocol 4A3MP | 4-Amino-M-Cresol |
| Rodol RS | Resorcinol |
| Rodol ERN | 1-Naphthol |
| Imexine OAG | 2-Methyl-5-Hydroxyethylaminophenol |
| Colorex WP5 | 1-Hydroxyethyl 4,5-Diamino Pyrazole Sulfate |
| Rodol PAOC | 4-Amino-2-Hydroxytoluene |
| HC Blue A42 | 2,4-Diaminophenoxyethanol di HCl |

**Tönungsgel**

| **Rohstoff** | **Rezeptur Nr. (Menge in Gew.-%)** | | | | | | | |
|---|---|---|---|---|---|---|---|---|
| | 1 | 2 | 3 | 4 | 5 | 6 | 7 | 8 |
| Wasser, demin. | 87,80 | 92,40 | 89,10 | 91,10 | 90,30 | 83,10 | 94,40 | 85,70 |
| Natrosol 250 HHX pharm | 1,5 | | 1,5 | | | | 2,0 | |
| Aristoflex AVC | | | | 2,0 | | | | |
| Gelcarin, Type GP 379 | | | | | | 2,0 | | |
| Grindsted Guar 178 | | | | | | 1,0 | | |
| Keltrol RD | | | | | 2,0 | | | 1,5 |
| Carbopol 981 | | 0,7 | | | | 0,4 | | |
| Citronensäure | 1,2 | | | | 1,5 | | | 1,5 |
| Phosphorsäure | | 1,0 | 2,0 | 1,5 | | 2,0 | 2,2 | |
| Ammoniaklösung 25% | | | 4,0 | 3,0 | 3,5 | | | 2,0 |
| Natriumhydroxid | 2,0 | | | | | 2,5 | | |
| Ethanolamin | | 4,0 | | | | | | |
| Dehyquart A-CA | 0,1 | | | | | 0,2 | | 0,2 |
| Euxyl K 300 | 1,0 | | 1,0 | 1,0 | 1,0 | | | |
| Neolone 950 | | 0,1 | | | | 0,1 | | 0,2 |
| Propylenglycol | 5,0 | | | | | 5 | 7 | 7 |
| Tego-Betain F 50 | | | | | 0,5 | | | |
| Euroxide LO | | | 0,5 | | | 1 | | 1,0 |
| Jellner QH 200 | | | | | | | 0,5 | |
| Texapon N70 | | | | 0,2 | | | 0,1 | |
| Glucquat 125 | | | 0,4 | | | 0,5 | | |
| C Sorbidex NC 16225 | | | | 0,3 | | | | |
| Volpo CS (B) ET2687 | 0,3 | 0,3 | 0,3 | 0,3 | 0,3 | 0,3 | | |
| Parfüm | 0,3 | 0,3 | 0,3 | 0,3 | 0,3 | 0,3 | 0,3 | |
| EDETA BD | 0,1 | | | | | 0,1 | 0,2 | 0,1 |
| Farbstoff A5 | | 0,5 | | 0,1 | 0,1 | 0,5 | | |
| Farbstoff A11 | 0,5 | 0,3 | 0,5 | 0,5 | | 0,3 | | |
| Farbstoff A23 | 0,2 | | 0,2 | 0,2 | | | | |
| Farbstoff A27 | | 0,4 | 0,2 | | 0,5 | 0,4 | | |
| Farbstoff A34 | | | | | | | 0,4 | |
| Arianor Steelblue | | | | | | 0,3 | 0,3 | |
| Arianor Sienna Brown | | | | | | | | 0,3 |
| Acid Violet 43 | | | | | | | 0,2 | |
| HC Blue 2 | | | | | | | | 0,5 |
| Patent Blue V | | | | | | | 0,1 | |
| Vibracolor Citrus Yellow | | | | | | | 0,5 | |

Wasser wurde vorgelegt und die Rezepturbestandteile nacheinander unter Rühren zugegeben. Vor dem Färben wurden die Rezepturen mindestens 1 Tag ruhen gelassen.

Jeweils 5 g einer Rezeptur wurden auf je eine 2 g Haarsträhne (gebleicht, Kerling) aufgebracht und 20 min einwirken gelassen. Anschließend wurde abgespült und getrocknet.

Folgende Färbeergebnisse wurden erzielt:

| **Rezeptur** | **Farbe** |
|---|---|
| 1 | braun |
| 2 | dunkelrot |
| 3 | goldbraun |
| 4 | rotbraun |
| 5 | kupfer |
| 6 | dunkelbraun |
| 7 | rotbraun |

**Tönungsemulsion**

| **Rohstoff** | **Rezeptur Nr. (Menge in Gew.-%)** | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| Wasser, de- min. | 65,20 | 68,30 | 66,50 | 63,90 | 65,60 | 63,70 | 63,80 | 64,90 | 64,80 | 65,70 | 64,25 | 64,10 |
| Lanette O | 7 | 5 | 10 | 8 | 8 | 8 | 8 | 8 | 8 | 8 | 8 | 8 |
| Volpo CS 20 (B) ET2687 | 0,1 | | | 0,1 | 0,1 | 0,1 | 0,1 | 0,1 | 0,1 | 0,1 | 0,1 | 0,1 |
| Super Harto- lan B | 2,0 | | | 2,0 | 2,0 | 2,0 | 2,0 | 2,0 | 2,0 | 2,0 | 2,0 | 2,0 |
| Eumulgin B1 | 0.5 | | | 0.5 | 0.5 | 0.5 | 0.5 | 0.5 | 0.5 | 0.5 | 0.5 | 0.5 |
| Dehydol LS 3 Deo N | | | 5 | | | | | | | | | |
| Sabosorb MLE | | | 3 | | | | | | | | | |
| Comperlan 100 | 1,0 | 3 | | 1,0 | 1,0 | 1,0 | 1,0 | 1,0 | 1,0 | 1,0 | 1,0 | 1,0 |
| Eutanol G | 5 | | | 5 | 5 | 5 | 5 | 5 | 5 | 5 | 5 | 5 |
| Ondina 917 | | 1 | | | | | | | | | | |
| Avocadoöl | | 1 | | | | | | | | | | |
| Saboderm DO | | | 2 | | | | | | | | | |
| Tegin VS | | 3 | 5 | | | | | | | | | |
| Dow Corning 949 | | | 0,5 | | | | | | | | | |
| Euroxide LO | 1,0 | | | | 1 | 1 | 1 | 1 | 1 | 1 | 1 | 1 |
| Tegobetain F 50 | | 1,0 | | | | | | | | | | |
| Texapon ALS/IS | | | 0,3 | | | | | | | | | |
| Phosphorsäu- re | 2,0 | | | | 2 | 2 | 2 | 2 | 2 | 2 | 2 | 2 |
| Citronensäu- re | | 2,0 | | 1 | | | | | | | | |
| Monoethano- lamin | | | | | 3 | | | | | | | |
| Natrium- hydroxid | | 2,1 | | | | | | | | | | |
| Ammoniaklö- sung 25% | 3,4 | | | | | 4,4 | 4,4 | 4,4 | 4,4 | 4,4 | 4,4 | 4,4 |
| Triethanola- min | | | | 6 | | | | | | | | |
| Natriumcar- bonat | | | 0,5 | | | | | | | | | |
| Timica Silver Sparkle | | 1,0 | | | | 1,0 | | | | | | |
| Glucquat 125 | 1,0 | | | | | | | | | | | |
| Crosilk Prote- in Komplex | 0,3 | | | | | | | | | | | |
| Nutrilan Kera- tin W | | 0,5 | | | | | | | | | | |
| EDETA BD | | 0,1 | 0,1 | 0,1 | 0,1 | 0,1 | 0,1 | 0,1 | 0,1 | 0,1 | 0,1 | 0,1 |
| Turpinal SL | 0,3 | | | | | | | | | | | |
| Glycerin | | 10 | 5 | | | | | | | | | |
| Propylengly- col | 5 | | | 5 | 5 | 5 | 5 | 5 | 5 | 5 | 5 | 5 |
| Natrosol 250 HHX Pharm. | 0,4 | 0,2 | | 0,3 | 0,3 | 0,3 | 0,3 | 0,3 | 0,3 | 0,3 | 0,3 | 0,3 |
| Carbopol 981 | | 0,2 | 0,4 | | | | | | | | | |
| Farbstoff A9 R | | | | | | 0,6 | | | | | | |
| Farbstoff A7 R | | | 0,7 | | | | | | | | | |
| Farbstoff A2 R | | | | 0,7 | | | | | | | | |
| Farbstof A36 R | | | | | 0,7 | | | | | | | |
| Farbstoff A12 V | 0,5 | 0,5 | 0,3 | | 0,5 | 0,2 | | | 0,4 | | | 0,7 |
| Farbstoff A15 R | | | | | | | | 0,7 | | | | |
| Farbstoff A23 O | 0,2 | 0,2 | 0,2 | | 0,3 | 0,4 | | | | | | |
| Farbstoff A28 Y | 0,1 | | | | | | | | 0,8 | | 0,3 | |
| Farbstoff A33 R | | | | | | | 0,7 | | | | 0,5 | |
| Arianor Steelblue | | 0,4 | | | | | | | | | | |
| Arianor Ma- hogany | | 0,3 | | | | | | | | | | |
| Arianor Sien- na brown | | | | 0,3 | | | | | | | | |
| HC Blue 2 | | | | 0,8 | | | 0,6 | | | | | |
| HC Violet BS | | | | 0,6 | | | | | | | | |
| Vibracolor Ruby Red | | | 0,2 | | | | | | | | | |
| HC Red 3 | | | | | | | | | | | | 0,5 |
| NHEAP | | | | | | | | | | | | 0,4 |
| Rodol 9R | | | | | | | 0,3 | | | | | 0,1 |
| Arianor Mad- er Red | | | | | | | | | | | 0,05 | |
| Arianor Straw Yellow | | | | | | | | | | | 0,2 | |
| Vibracolor Citrus Yellow | | | | | | | | | | | 0,3 | |
| Vibracolor Flame Oran- ge | | | | | | | | | | | 0,2 | |
| Patent Blue V | | | | | | | 0,1 | | | | | |
| Brilliant Blue | | | | | | | | | | 0,1 | | |
| Azorubin | | | | | | | 0,1 | | | | | |
| Acid Violet 43 | 0,2 | | | | | | | | | 0,1 | | |
| Henna Lea- ves | | | | | | | 0,3 | | | | | |
| Parfüm | 0,3 | 0,2 | 0,3 | 0,2 | 0,4 | 0,2 | 0,2 | 0,5 | 0,1 | 0,2 | 0,3 | 0,3 |

Die Emulsionen wurden nach folgender Vorschrift hergestellt:
Die Fettphase wurde auf 70°C erwärmt. Wasser wurde auf 75°C erwärmt und die wasserlöslichen Komponenten sowie die Farbstoffe zugefügt. Die Phasen wurden vereinigt. Es wurde homogenisiert und nach dem Abkühlen die empfindlichen Wirkstoffe und das Parfüm eingerührt.

Die Emulsionen wurden in verschiedene Kunststofftuben (Polyethylen, Polypropylen, Polyamid und Polyvinylchlorid) mit unterschiedlichen Öffnungen (Auftragespitze, Membranverschluss) sowie in Aluminiumtuben gefüllt.

Nach einer Ruhezeit von 1 Tag wurde ausgefärbt. Dazu wurden 5 g Emulsion auf eine 2 g Haarsträhne (Imhair 90 % grau) aufgetragen und nach 20 min ausgespült und getrocknet.

| **Rezeptur** | **Farbergebnis** |
|---|---|
| 1 | Goldbraun |
| 2 | Dunkelbraun |
| 3 | Rot |
| 4 | Rotbraun |
| 5 | Dunkelrot |
| 6 | Rot |
| 7 | Rotbraun |
| 8 | Rot |
| 9 | Braun |
| 10 | Kupfer |
| 11 | Dunkelrot |

Die Emulsionen 2 und 9 wurden ebenfalls auf Wimpern ausgefärbt, die Einwirkzeit betrug hierbei 3 min. Die Farbergebnisse fielen ähnlich aus wie auf den grauen Haaren aus. Der Farbton war allerdings aufgrund der Eigenfarbe der Wimpern jeweils dunkler.

Mit den Emulsionen 3, 5 und 10 wurden Finger- und Fußnägel gefärbt. Dazu wurde eine Schicht der Emulsion mit einem Pinsel aufgetragen und 5 min auf den Nägeln belassen. Nach dem Abspülen waren die Nägel intensiv gefärbt.

**Schaumtönung**

| **Rohstoff** | **Gew.-%** |
|---|---|
| Wasser demin. | 43,1 |
| Ethanol | 12,3 |
| Monoethanolamin | 0,2 |
| Eutanol G | 5,0 |
| Texapon ALS/IS | 2,0 |
| Tego Betain F 50 | 12 |
| EDETA BD | 0,1 |
| Parfüm | 0,5 |
| Farbstoff A31 | 1,0 |
| Propan/Butan | 10 |

Die Komponenten wurden nacheinander in Wasser gelöst, in eine Aluminiumaerosoldose gefüllt und mit der angegebenen Menge Treibgas beaufschlagt. Nach 1 Tag Ruhezeit wurden 2 g Schaum auf eine Haarsträhne (Imhair 90 % grau) aufgetragen. Nach 20 min Einwirkzeit wurde abgespült und getrocknet. Die Strähne zeigte eine intensive rote Farbe.

**Färbendes Wimperngel**

| **Rezeptur** | **Gew.-%** |
|---|---|
| Wasser, demin. | 68,4 |
| Glycerin | 5 |
| Pluracol E 8000 | 5 |
| Dow Corning 245 Fluid | 3 |
| Natriumhydroxid | 0,5 |
| 1,3 Butylene Glycol | 6 |
| Carbopol 981 | 1 |
| Celquat L-200 | 0,2 |
| Luviskol VA 64W | 10 |
| EDETA BD | 0,1 |
| Euxyl K300 | 0,3 |
| Farbstoff A11 | 0,2 |
| Farbstoff A26 | 0,3 |

Herstellung: Verdicker Carbomer bei ca. 60°C in der Wasserphase quellen lassen, unterrühren abkühlen, Natriumhydroxid zugeben, Farbstoffe lösen, Öle einrühren.

Das Gel wurde auf Wimpern aufgetragen und erzielte einen direkten braunen Farbeffekt. Auch nach dem Entfernen war noch ein Farbeffekt sichtbar. Bei wiederholter Anwendung verstärkte sich der Effekt. Das Gel ist auch hervorragend zum gleichzeitigen Festigen und Färben von Haupthaar geeignet.

**Färbende Blondiermittel**

| **Rohstoff** | **Rezeptur Nr (Menge in Gew.-%)** | |
|---|---|---|
| | **1** | **2** |
| Magnesia 10 | 8,7 | 12,1 |
| Kaliumperoxodisulfat | 30 | 30 |
| Ammoniumperoxodisulfat | 6 | 6 |
| Natrosol 250 HHX | 0,4 | 0,4 |
| EDTA BD | 1 | 1 |
| Texapon Z95P | 0,1 | 0,1 |
| Aerosil 200 | 2 | 2 |
| Eutanol G | 7 | |
| Portil A | 40 | 27 |
| Ondina 917 | 3 | 20 |
| Parfüm | 0,3 | 0,2 |
| Jellner QH-200 | 0,2 | |
| Farbstoff A9 | 0,7 | |
| Farbstoff A12 | 0,3 | |
| Farbstoff A26 | 0,3 | 0,6 |
| Farbstoff A34 | | 0,6 |

Die festen Komponenten werden in einem Pulvermischer mit Messern vermengt und anschließend mit der Mischung der flüssigen Komponenten verrührt.

5 g des jeweiligen Blondiermittels wurden mit 6%iger wässriger Wasserstoffperoxidlösung gemischt und auf eine 2 g Naturhaarsträhne (Kerling) appliziert. Nach 30 min Einwirkzeit wurde die Strähne gespült und getrocknet. Die Haarsträhnen waren wie folgt gefärbt:

| **Rezeptur** | **Farbergebnis** |
|---|---|
| 1 | Hellrot |
| 2 | Intensiv Kupfer |

**Farbshampoo**

| **Rohstoff** | **Rezeptur Nr. (Menge in Gew.-%)** | |
|---|---|---|
| | **1** | **2** |
| Wasser demin. | 56,3 | 75,3 |
| Texapon N70 | 14 | 10 |
| Texapon ALS/IS | | 6 |
| Tego-Betain F50 | 4 | 1 |
| Miranol Ultra C-32 | 8 | |
| Lamesoft PO 65 | 2 | |
| Jellner QH 200 | 0,2 | 0,4 |
| Salcare Super 7 | 2 | |
| Jaguar Excel | 0,3 | |
| Propylenglycol | 1 | |
| Nutrilan Keratin W | | 0,5 |
| Dehydol LS 3 | | 1 |
| Phosphorsäure | 0,6 | 1,0 |
| Natriumhydroxid | 0,4 | 0,7 |
| Parfüm | 0,3 | |
| Uvinol DS 49 | 0,5 | |
| Euperlan PK 900 Benz-W | 8 | |
| Lanesoft TM Benz | | 5 |
| DL-Panthenol | 1,0 | |
| Natriumchlorid | 0,5 | 0,7 |
| Phenoxyethanol | 0,3 | |
| Benzylalkohol | 0,3 | |
| Neolone 950 | 0,1 | |
| Oxtopirox | | 0,3 |
| Farbstoff A19 | 0,2 | |
| Farbstoff A12 | | 0,5 |

Die Komponenten wurden nacheinander in Wasser gelöst. Durch Zugabe von Natriumchlorid wurde die Viskosität eingestellt.

Je 3 g der Farbshampoos wurden auf eine 2 g Humanhaarsträhne (Kerling 90 % grau) appliziert. Nach 5 min Einwirkzeit wurde die Strähne gespült und getrocknet. Die Haarsträhnen waren wie folgt gefärbt:

| **Rezeptur** | **Farbergebnis** |
|---|---|
| 1 | rot |
| 2 | violett |

**Farbconditioner**

| **Rohstoff** | **Rezeptur Nr. (Menge in Gew.-%)** | | | |
|---|---|---|---|---|
| | **1** | **2** | **3** | **4** |
| Wasser demin. | 74,67 | 85,2 | 80,6 | 81,25 |
| Lanette O | 9 | 7 | 4 | 5 |
| Kakaobutter | | | 4 | |
| Genamin KDMP | | 1,0 | 1,0 | 1,0 |
| Dehyquart A | | 0,5 | | 0,5 |
| Tego Amid S 18 | | 2 | | |
| Eutanol G | 2 | | 2 | 1,0 |
| Saboderm DO | | | | 0,5 |
| Volpo CS 20 (B) ET 2687 | 0,1 | 0,1 | | |
| Eumulgin B1 | 0,9 | | 0,8 | |
| Avocado Oil | 2,0 | | | |
| Natriumhydroxid | 0,25 | 0,4 | 0,4 | 0,5 |
| Phosphorsäure 85% | 0,33 | 0,5 | 0,5 | 0,5 |
| Propylenglycol | 5,0 | 2,0 | 4,0 | 3,0 |
| Natrosol 250 HHX pharm | 0,3 | | | |
| Euxyl K 300 | 1,0 | 1,0 | 1,0 | 1,0 |
| Luviquat FC 370 | 0,5 | | | |
| Silwet Copolymer L-7600 | | | 0,2 | |
| Dow Corning 939 | | | 0,5 | |
| Dow Corning 246 Fluid | | | | 0,5 |
| Gafquat 755N-PW | | | | 5,0 |
| Crosilk Protein Komplex | | | 0,5 | |
| Crotein Q | 3 | | | |
| DL-Panthenol | 0,2 | | | |
| Parfüm | 0,5 | 0,2 | 0,3 | 0,2 |
| Farbstoff A15 | 0,1 | 0,1 | 0,1 | |
| Farbstoff A24 | 0,05 | | 0,1 | |
| Farbstoff A11 | 0,1 | | | 0,05 |

Die Conditioner wurden nach folgender Vorschrift hergestellt:
Die Fettphase wurde auf 70°C erwärmt. Wasser wurde auf 75°C erwärmt und die wasserlöslichen Komponenten sowie die Farbstoffe zugefügt. Die Phasen wurden vereinigt. Es wurde homogenisiert und nach dem Abkühlen die empfindlichen Wirkstoffe und das Parfüm eingerührt.

Je 3 g der Conditioner wurden auf eine 2 g Humanhaarsträhne (Kerling 2 h gebleicht) appliziert. Nach 5 min Einwirkzeit wurde die Strähne gespült und getrocknet. Die Formulierungen zeichnen sich dadurch aus, dass die neben einem hervorragenden Pflegeeffekt noch die Farbe der Haare ändern. Die Haarsträhnen waren wie folgt gefärbt:

| **Rezeptur** | **Farbergebnis** |
|---|---|
| 1 | Braun |
| 2 | Rot |
| 3 | Kupfer |
| 4 | violett |

**Farbschaum**

| **Rohstoff** | **Rezeptur Nr. (Menge in Gew.-%)** | | | |
|---|---|---|---|---|
| | **1** | **2** | **3** | **4** |
| Wasser demin. | 65,8 | 75,5 | 75,6 | 60,6 |
| Ethanol | | | | 15 |
| Luviquat Supreme | | 7,0 | 3 | 3 |
| Silwet Copolymer L-7600 | | | 0,3 | 0,3 |
| Cellquat L-200 | | | | 0,2 |
| Dehyquart A-CA | | 1,0 | | |
| Panthenol | | 0,1 | 0,1 | 0,1 |
| Isopropanol | 20,0 | | | |
| Luviskol VA 64 W | 2,0 | 5,0 | 9,0 | 9,0 |
| Gafquat 755N-PW | 1,0 | | | |
| Luviquat Mono CP | | 0,5 | | |
| Luviquat FC 390 | | | 0,5 | 0,5 |
| Citronensäure | | 0,1 | 0,2 | 0,2 |
| Eumulgin B1 | 0,5 | | | |
| Parfüm | 0,3 | 0,2 | 0,3 | 0,3 |
| Chremophor RH 40 | | 0,2 | 0,2 | 0,2 |
| Natriumbenzoat | | 0,1 | 0,2 | 0,2 |
| Euxyl K 300 | 0,2 | | | |
| Uvinul A Plus | 0,2 | | | |
| Solamer GR8 | | | 0,2 | |
| Uvinol DS 49 | | | 0,1 | 0,1 |
| Propan/Butan | 10 | 10 | | |
| Dimethylether | | | 10 | |
| Distickstoffmonoxid | | | | 10 |
| Farbstoff A21 | | 0,3 | 0,3 | 0,3 |

100 g der Rezeptur wurden in eine Aerosoldose eingefüllt und nach dem Verschließen mit dem Treibgas beaufschlagt. 2 g des Schaumes wurden auf eine Haarsträhne appliziert. Die Strähne wurde mit einem Fön getrocknet. Die Formulierungen besitzen gleichzeitig eine rot färbende und festigende Wirkung.

**Fönfestiger**

| **Rohstoff** | **Gew.-%** |
|---|---|
| Wasser demin. | 58,35 |
| Ethanol | 40 |
| Luviskol VA 64W | 0,4 |
| Gafquat 755N-PW | 0,3 |
| Dow Corning 939 | 0,5 |
| Cremophor RH 40 | 0,2 |
| Parfüm | 0,2 |
| Farbstoff A12 | 0,05 |

0,3 g des Fönfestigers werden auf eine Haarsträhne (Kerling 80 % grau) aufgetragen. Die Strähne wurde trocken gefönt. Der ursprünglich gelbliche Farbton der Haarsträhne war weitgehend neutralisiert. Der Effekt war auch nach 3 Haarwäschen noch erkennbar.

**Oxidative Haarfärbemittel**

| **Rohstoff** | **Rezeptur Nr. (Menge in Gew.-%)** | | | |
|---|---|---|---|---|
| | **1** | **2** | **3** | **4** |
| Wasser demin. | 57,2 | 64,6 | 67,2 | 60,7 |
| Lanette O | 12 | 12 | 10 | 13 |
| Eutanol G | 3 | | 1,0 | |
| Comperlan 100 | 1,5 | | 2 | 2 |
| Tegin VS | | | 5 | |
| Super Hartolan B | 3,0 | 2,5 | | 2,5 |
| Avocadoöl | 1,5 | | | 2 |
| Fitoderm | | | 1,0 | |
| Meadowfoam Seed Oil | | 1,0 | | |
| Pristerene 4960 | 6 | 5,5 | | 6 |
| Coenzyme Q 10 | | | 0,1 | |
| EDTA BD | 0,1 | 0,1 | 0,1 | 0,1 |
| Texapon K12G | 0,5 | | 0,1 | 0,1 |
| Propylenglycol | 5 | | 5 | 5 |
| Glycerin | | 5 | | |
| Texapon N70 | | 2,0 | | |
| Timica Silver Sparkle | 1,0 | | | |
| Monoethanolamin | | 4 | | |
| Ammoniaklösung 25% | 6 | | 3 | 6 |
| 2,5-Diaminotoluolsulfat | 1,4 | 0,9 | | 1,3 |
| Rodol RS | 0,3 | 0,2 | | 0,3 |
| Rodol ERN | | | 0,3 | |
| Imexine OAG | | | 1,0 | |
| Colorex WP5 | | | 2,6 | |
| HC Blue A42 | 0,1 | | | |
| Jarocol 4A3MP | | 0,6 | | |
| Rodol PAOC | | 0,5 | | |
| Natriumsulfit | 0,5 | | | |
| Uantox EBATE | | 0,5 | | |
| Ascorbinsäure | | | 0,3 | |
| Covastyle TBQ | | | | 0,3 |
| Farbstoff A10 | | 0,4 | | 0,5 |
| Farbstoff A23 | | | 0,4 | |
| Farbstoff A12 | 0,7 | | | |
| Farbstoff A25 | | | 0,6 | |
| Parfüm | 0,2 | 0,2 | 0,3 | 0,2 |

Die Fettkomponenten wurden auf 80°C erhitzt. Die wasserlöslichen Komponenten wurden in 80°C warmes Wasser gegeben und gelöst. Die Phasen wurden vereinigt und homogenisiert. Nach dem Abkühlen wurde das Parfüm zugegeben. Die Cremes wurden sofort in Aluminiumtuben, in PVC Tuben sowie in coextrudierte Tuben aus Polyethylen mit einer Barriereschicht gefüllt, die den Inhalt vor Kontakt mit Sauerstoff schützt. Einige zwei Komponentenpackmittel wurden derart befüllt, dass in eine Kammer die Farbcreme und in die andere eine Wasserstoffperoxidlösung gefüllt wurde. Vor der Anwendung werden diese beiden Komponenten nach Durchbrechen einer Barriere durch Schütteln gemischt.

Jeweils 5 g der oxidativen Haarfärbemittel wurden mit 6%iger Wasserstoffperoxidlösung gemischt und auf eine 2 g Naturhaarsträhne (Kerling) appliziert. Nach 30 min Einwirkzeit wurde die Strähne gespült und getrocknet. Die Haarsträhnen waren wie folgt gefärbt:

| **Rezeptur** | **Farbergebnis** |
|---|---|
| 1 | braun-violett |
| 2 | rotbraun |
| 3 | intensives rot |
| 4 | braun-rot |

**Haarwasser**

| **Rohstoff** | **Rezeptur Nr. (Menge in Gew.-%)** | | | |
|---|---|---|---|---|
| | **1** | **2** | **3** | **4** |
| Wasser | 57,69 | 69,18 | 59,18 | 84,29 |
| Ethanol | 40 | | 20 | 15 |
| Isopropanol | | 30 | 20 | |
| Cremophor RH 40 | 0,2 | 0,2 | 0,3 | 0,2 |
| Parfüm | 0,3 | 0,2 | 0,3 | 0,2 |
| Menthol | 0,4 | | 0,1 | |
| Coffein | 0,3 | | 0,1 | |
| Hopfen-Extrakt HS 2367G | 0,3 | | | |
| DL-Panthenol | 0,5 | | | |
| Biotin | | 0,1 | | |
| Parfüm | 0,3 | 0,3 | | 0,3 |
| Farbstoff A11 | 0,01 | 0,02 | | |
| Farbstoff A12 | | | 0,02 | 0,01 |

Die Haarwässer wurden in PET-Flaschen mit Auftragespitze gefüllt.
Je 1 g der Rezepturen wurden auf eine blondierte Haarsträhne und eine graumelierte Haarsträhne (90 % grau, Kerling) aufgebracht und einmassiert. Die Strähnen wurden getrocknet. Bei allen Strähnen zeigt sich, dass der Gelbanteil in der Farbe der Strähnen deutlich reduziert wurde.

**Mascara**

| **Rohstoff** | **Rezeptur Nr. (Menge in Gew.-%)** | | |
|---|---|---|---|
| | **1** | **2** | **3** |
| Wasser | 52,36 | 67,34 | 72,37 |
| Pristerene 4960 | 6 | 5 | 5,5 |
| Bienenwachs weiss | 5 | | 2 |
| Carnauba Wax 2442 | 5 | 3 | 4 |
| Lunacera C 40 | 4 | 3 | |
| Lunacera MWN (Paramelt) | | 1 | |
| Kester Wachs K82H | | | 5 |
| Dow Corning 245 Fluid | 5 | | |
| Silfoam SE 2 | 1 | 1 | |
| Glycerin | 3 | | 5 |
| 1,3 Butylene Glycol | | 2 | |
| Resyn XP | 2 | 5 | 3 |
| Antaron V-220 | 2 | | |
| Gummi Arabicum | | | 0,5 |
| Triethanolamine | 2 | 2 | 2 |
| Ariabel Black | 10 | 5 | |
| C61-1245 SunCROMA Chrome Oxide Green | | 5 | |
| Luzenac Pharma 30A | 2 | | |
| Euxyl K 300 | 0,5 | 0,5 | 0,5 |
| Farbstoff A12 | 0,10 | | |
| Farbstoff A11 | | 0,10 | 0,1 |
| Farbstoff A28 | 0,03 | 0,04 | 0,02 |
| Farbstoff A 29 | 0,01 | | 0,01 |
| Farbstoff A 32 | | 0,02 | |

Die Rezepturen wurden nach folgender Vorschrift hergestellt:
Fettphase bei ca. 90°C aufschmelzen, Wasserphase auf ca. 80°C erwärmen, Neutralisationsmittel, Farbstoffe und Filmbildner zugeben und lösen, bei 80°C Fettphase und Wasserphase emulgieren, unter Rühren abkühlen lassen.

Mit Hilfe einer für Mascaras üblichen Bürste wurde die Rezeptur auf die Wimpern aufgetragen. Nach dem Entfernen der Rezeptur von den Wimpern waren diese dunkler gefärbt als vor der Anwendung. Durch wiederholte Anwendung verstärkte sich der Effekt.

## Patentansprüche

1. Verwendung wenigstens einer der Verbindungen der Formeln 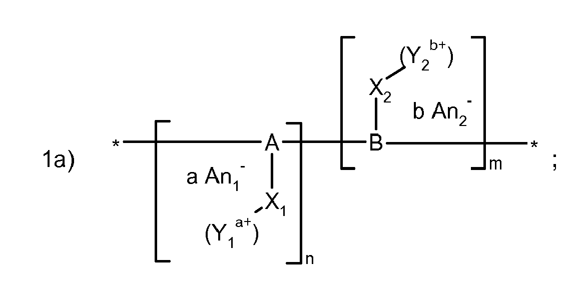 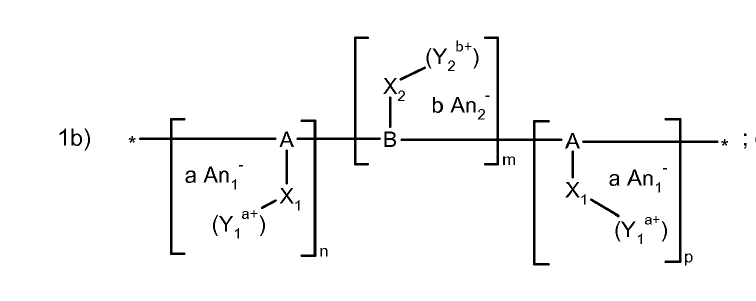 oder 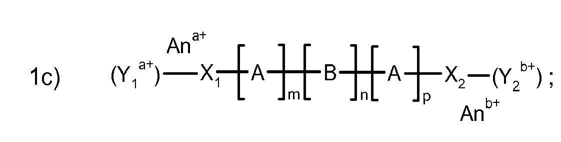 obei
A und B,unabhängig voneinander eine Polymerkette darstellen;
X₁ und X₂ unabhängig voneinander eine Verbindungsgruppe ausgewählt aus-C₁-C₁₀Alkylen; -C₂-A-; -C₅-₋C-; -C₅-A-, -C₅-Arylen-(C₁-C₁₀alkylen)-; -C(O)-; -(CH₂CH₂-O)₁₋₅-; -C(O)O-; -OCO-; -N(R₁)-; -CON(R₁)-; -(R₁)NC(O)-; -O-; -S-; -S(O)-; -S(O)₂-; -S(O)₂-N(R₁R₂); oder einer direkten Bindung;
R₁ und R₂ unabhängig voneinander Wasserstoff oder eine unsubstituierte oder Substituierte, geradkettiger oder verzweigte, unabhängig, monocyclische oder polycyclische, unterbrochene oder ununterbrochene C₁-C₄Alkyl; C₂-C₁₄Alkenyl; C₆-C₁₀Aryl; C₆-C₁₀Aryl-C₁-C₁₀alkyl; or C₅-C₁₀Alkyl(C₅-C₁₀aryl) darstellen
Y₁ und Y₂ unabhängig voneinander unabhängigeine organische Chromophorgruppe oder Wasserstoff darstellen, wobei mindestens eine der Gruppen Y₁ und Y₂ eine organische Chromophorgruppe ist;
An₁, An₂ und An₃, unabhängig voneinander unabhängigein Anion darstellen;
a und b unabhängig voneinander unabhängigeine Zahl von 1 bis 3 ist; m eine Zahl von 0 bis 1000 ist n eine Zahl von 0 bis 1000 ist p eine Zahl von 0 bis 1000 ist
wobei die Summe m + n + p ≥ 3 ist.
in einem Kosmetikprodukt zur Färbung von Haaren, insbesondere zur Färbung von menschlichem Kopfhaar, Augenbrauen, Wimpern, Körperbehaarung und/oder Bärten.

2. Verbindung wie in Anspruch 1 beschrieben, wobei Y₁ und Y₂ unabhängig voneinander gewählt werden unter Anthrachinon-, Azo-, Azomethin-, Hydrazomethin-, Merocyanin-, Methin- and Styrylchromophorgruppen.

3. Verwendung nach Patentanspruch 1, **dadurch gekennzeichnet, dass** die Verbindungen A1 bis A30, A32 bis A36 eingesetzt werden.

4. Verwendung nach Patentanspruch 1 oder 2, **dadurch gekennzeichnet, dass** in dem Kosmetikprodukt zusätzlich Direktfarbstoffe enthalten sind.

5. Verwendung nach einem der vorangehenden Patentansprüche, **dadurch gekennzeichnet, dass** in dem Kosmetikprodukt zusätzlich oxidative Farbstoffe enthalten sind.

6. Verwendung nach einem der vorangehenden Patentansprüche, **dadurch gekennzeichnet, dass** in dem Kosmetikprodukt zusätzlich Pigmente enthalten sind.

7. Verwendung nach einem der vorangehenden Patentansprüche, **dadurch gekennzeichnet, dass** in dem Kosmetikprodukt zusätzlich alkalische Streckmittel enthalten sind.

8. Verwendung nach einem der vorangehenden Patentansprüche, **dadurch gekennzeichnet, dass** in dem Kosmetikprodukt zusätzlich aufhellende Mittel enthalten sind.

9. Verwendung nach einem der vorangehenden Patentansprüche, **dadurch gekennzeichnet, dass** in dem Kosmetikprodukt zusätzlich reduzierende Wellmittel enthalten sind.

10. Verwendung nach einem der vorangehenden Patentansprüche, **dadurch gekennzeichnet, dass** in dem Kosmetikprodukt das Produkt als Gel, Emulsion, Paste oder pulverförmige Zubereitung vorliegt.

11. Verwendung nach einem der vorangehenden Patentansprüche, **dadurch gekennzeichnet, dass** das Produkt in Tuben, Flaschen, Beutel, Dosen verpackt vorliegt.

12. Verfahren zur Färbung von Haaren umfassend die Schritte
a) Behandlung des Haars mit einem aufhellenden Mittel,
b) bedarfsfalls Behandlung des Haars mit einem reduzierenden Wellmittel,
c) Behandlung des Haars mit einem Kosmetikprodukt nach einem der Patentansprüche 1 bis 6.

13. Verfahren nach Anspruch 12, **dadurch gekennzeichnet, dass** es die Färbung von menschlichem Kopfhaar, Augenbrauen, Wimpern, Körperbehaarung und/oder Bärten betrifft.
